# EUROPEAN PATENT SPECIFICATION

(11) **EP 3 996 710 B1**
(45) Date of publication and mention of the grant of the patent: **22.01.2025**
(21) Application number: 20739952.8
(22) Date of filing: 09.07.2020
(51) Int. Cl.: A61K 31/437, A61K 31/138, A61K 31/337, A61K 31/4166, A61K 31/517, A61K 31/704, A61K 33/24, A61K 33/243, A61K 45/06, A61P 35/00

(54) **INHIBITOR OF MAP KINASE INTERACTING SERINE/THREONINE KINASE 1 (MNK1) AND MAP KINASE INTERACTING SERINE/THREONINE KINASE 2 (MNK2), CANCER THERAPY AND THERAPEUTIC COMBINATIONS**
INHIBITOR DER MAP-KINASE-INTERAGIERENDEN SERIN/THREONIN-KINASE 1 (MNK1) UND DER MAP-KINASE-INTERAGIERENDEN SERIN/THREONIN-KINASE 2 (MNK2), KREBSTHERAPIE UND THERAPEUTISCHE KOMBINATIONEN
INHIBITEUR DE SÉRINE/THRÉONINE KINASE 1 INTERAGISSANT AVEC UNE MAP KINASE (MNK1) ET SÉRINE/THRÉONINE KINASE 2 INTERAGISSANT AVEC UNE MAP KINASE (MNK2), THÉRAPIE ANTICANCÉREUSE ET COMBINAISONS THÉRAPEUTIQUES

(30) Priority: 10.07.2019 ES 201930643
(43) Date of publication of application: 18.05.2022
(73) Proprietor: Fundació Hospital Universitari Vall d'Hebron - Institut de Recerca, 08035 Barcelona (ES); Institut Químic de Sarrià CETS Fundació Privada, 08017 Barcelona (ES); Consorcio Centro de Investigación Biomédica en Red, 28029 Madrid (ES)
(72) Inventor: RAMON Y CAJAL AGÜERAS, Santiago, 08035 BARCELONA (ES); HÜMMER, Stefan, 08035 BARCELONA (ES); CASTELLVI VIVES, Josep, 08035 BARCELONA (ES); MARTINEZ SÁEZ, Elena, 08035 BARCELONA (ES); BORRELL BILBAO, José Ignacio, 08017 BARCELONA (ES); TEIXIDÓ CLOSA, Jordi, 08017 BARCELONA (ES); ESTRADA TEJEDOR, Roger, 08017 BARCELONA (ES); BOU PETIT, Elisabeth, 08017 BARCELONA (ES); PEG CAMARA, Vicente, 08035 BARCELONA (ES); GUIJARRO CARRILLO, Pedro Jesus, 08035 BARCELONA (ES); SANTAMARIA MARGALEF, Anna, 08035 BARCELONA (ES); MOROTE ROBLES, Joan, 08173 SANT CUGAT DEL VALLÉS (ES); SUAREZ CABRERA, Leticia, 08035 BARCELONA (ES)
(74) Representative: ZBM Patents - Zea, Barlocci & Markvardsen
(86) International application number: PCT/EP2020/069459
(87) International publication number: WO 2021/005183

(56) References cited:
- WO-A1-2016/172010
- US-A1- 2010 113 415
- CHEL HUN CHOI ET AL: "Direct inhibition of eIF4E reduced cell growth in endometrial adenocarcinoma", JOURNAL OF CANCER RESEARCH AND CLINICAL ONCOLOGY, SPRINGER, BERLIN, DE, vol. 137, no. 3, 13 May 2010 (2010-05-13), pages 463 - 469, XP019881022, ISSN: 1432-1335, DOI: 10.1007/S00432-010-0902-Z
- HOU S ET AL: "Significance of MNK1 in prognostic prediction and chemotherapy development of epithelial ovarian cancer", CLINICAL AND TRANSLATIONAL ONCOLOGY, SPRINGER ITALIA SRL, ITALY, SPAIN, vol. 19, no. 9, 22 March 2017 (2017-03-22), pages 1107 - 1116, XP036293517, ISSN: 1699-048X, [retrieved on 20170322], DOI: 10.1007/S12094-017-1646-X
- LIU S ET AL: "Inhibiting ERK/Mnk/eIF4E broadly sensitizes ovarian cancer response to chemotherapy", CLINICAL AND TRANSLATIONAL ONCOLOGY, SPRINGER ITALIA SRL, ITALY, SPAIN, vol. 20, no. 3, 1 August 2017 (2017-08-01), pages 374 - 381, XP036442047, ISSN: 1699-048X, [retrieved on 20170801], DOI: 10.1007/S12094-017-1724-0
- VIDYA P. RAMAMURTHY ET AL: "Simultaneous targeting of androgen receptor (AR) and MAPK-interacting kinases (MNKs) by novel retinamides inhibits growth of human prostate cancer cell lines", ONCOTARGET, vol. 6, no. 5, 20 February 2015 (2015-02-20), pages 3195 - 3210, XP055735024, DOI: 10.18632/oncotarget.3084
- ELISABETH BOU PETIT: "Sensitizing triple negative breast cancer to approved therapies: Design, synthesis and biological activity of MNK inhibitors", TESIS DOCTORALS EN XARXA, 6 September 2019 (2019-09-06), pages 1 - 4, XP055734696, Retrieved from the Internet <URL:https://www.tdx.cat/handle/10803/667814> [retrieved on 20200929]

## Description

This application claims the benefit of Spanish Patent Application P201930643 filed on July 10, 2019.

### Technical Field

The present invention relates to 4,6-diphenyl-1*H*-pyrazolo[3,4-*b*]pyridin-3-amine, or of a pharmaceutically acceptable salt thereof, as a selective MNK inhibitor, for use in the treatment of a cancer of an hormone-dependent organ, particularly for use in the treatment of breast cancer, prostate cancer, ovarian cancer or endometrial cancer.

### Background Art

Breast cancer is the most common cancer in women worldwide and the second leading cause of cancer-related death. Despite global advances in cancer treatment, the fatality in some cancer subtypes with a poor prognosis has not improved substantially in recent years. Among the different breast cancer subtypes with a poor prognosis is triple-negative breast cancer (TNBC), characterised by the absence of estrogen receptor α (ER), progesterone receptor (PR) and human epidermal growth factor receptor 2 (HER-2) [Clin Cancer Res. 2007;13:4429-4434]. This cancer subtype commonly develops with a relatively aggressive histopathological phenotype and tends to be diagnosed at younger ages than in ER-positive cases [Nat Rev Clin Oncol. 2016;13:674-690; Oncologist. 2011;16 Suppl 1:1-11; Breast Cancer Res. 2010;12:212]. Since triple-negative tumours lack the primary targets of approved therapies, the cure thereof is currently limited to the application of surgery, radiotherapy and/or systemic chemotherapy. Therefore, despite advances in understanding the underlying biology of specific cancer subtypes, it is especially important to identify new therapeutic strategies for the treatment of these diseases.

Many studies have shown a significant clinical benefit of chemotherapy on TNBC in neoadjuvant, adjuvant and metastatic settings. Paradoxically, TNBC cases generally show better initial responses to chemotherapy than patients with other breast cancer subtypes [J. Am. Soc. Clin. Oncol. 2008;26:1275-1281]. However, complete pathological responses are only achieved in 30-40 % of cases at an early stage and patients who do not show this response level have a risk of death that is 12 times higher [Lancet Lond. Engl. 2014;384:164-172; J. Am. Soc. Clin. Oncol. 2012;30:1796-1804]. To improve the curing of cancer in this context, new strategies are being tested, such as immunotherapy, but its success to date is limited [Med. Oncol. Northwood Lond. Engl. 2017;35:13].

Other subtypes which are estrogen positive have a better prognosis but a high risk of late recurrences. In fact, patients are treated with antiestrogens for many years. (J Clin Oncol. 2015 Mar 10;33(8):916-22). In this sense, various combinatorial approaches are being tried to optimize, including the combination with mnk inhibitors (Genes Dev. 2017 Nov 15;31(22):2235-2249)

Another type of cancer that developes desentizitation to approved treatments over time is prostate cancer. Prostate cancer is the most common malignancy and the second cause of cancer-related deaths among men in the western countries [CA Cancer J Clin. 2020 Jan;70(1):7-30]. Most deaths occur after androgen-deprivation therapy failure, when the tumor evolves to castration resistant prostate cancer (CRPC). Despite initial response to second line anti-hormonal therapies (suchs as abiraterone and enzalutamide) patients usually develop resistance to these agents [BJU Int. 2016 Feb;117(2):215-25]. Intratumoral androgen production, amplification, mutation or expression of consitutively active AR splice variantes lacking the C-terminal ligand-binding domain (being AR-V7 one of the most commonly found in CRPC patients) are some of the most important clinical mechanisms of resistance to anti-hormonal therapies [Cancer Treat Rev. 2017 Jun; 57: 16-27]. As one of the main obstacles to the treatment of advanced prostate cancer is drug resistance, therapeutic combinatory strategies are desired.

Deregulation of protein synthesis is a common event in human cancer and, in particular, in resistance to chemotherapeutic agents. A key factor in translational control is the translation initiation factor 4E (eIF4E), the function of which is modulated by the MAP kinase-interacting serine/threonine-protein kinase 1, MNK1, and the MAP kinase-interacting serine/threonine-protein kinase 2, MNK2, through phosphorylation of a conserved serine (Ser209).

In recent years, elF4E has been described as an independent prognostic factor associated with malignant progression and adverse prognosis in various tumours, including breast cancer, lung cancer, ovarian cancer, endometrial cancer, glioma and prostate cancer [Clin Transl Oncol. 2014;16:937-1113]. Other groups have confirmed the prognostic importance of these factors in additional types of tumours, like colon cancer [Oncotarget. 2015;6:24092-24104], nasopharyngeal carcinoma [PLoS ONE. 2014;9:e89220], hepatocellular carcinoma [J Cancer Res Clin Oncol. 2016;142:2309-2317], astrocytomas [J Neurooncol. 2016;131:485-493], lung cancer [Virchows Arch. 2015;467:667-673; Int J Clin Exp Pathol. 2015;8:3955-3962] and melanoma [J Invest Dermatol. 2015;135:1358-1367]. The worst prognosis has been associated with increased epithelial-mesenchymal transition in cells with p-elF4E overexpression [Oncogene. 2015;34(16):2032-2042] and a greater resistance to cellular oxidative stress, lack of nutrients and anti-tumour agents [PLoS ONE. 2015;10(4):e0123352].

Studies have provided proof of concept that the deregulation of elF4E-mediated translation initiation is an important step in oncogenic transformation and may contribute to tumour maintenance. Although phosphorylation is necessary for oncogenic transformation, it appears to be dispensable for normal development. Therefore, pharmacological MNK inhibitors may present an effective, non-toxic anti-cancer strategy, especially in combination with approved treatments. In summary, MNK1/2 have emerged as targets of interest for drug discovery in oncology, based on the anti-tumour effects observed in the experiments using RNA interference and the absence of adverse effects in double-knockout animals [Mol Cell Biol. 2004;24(15):6539-6549]. However, the lack of selective MNK inhibitors has hampered the validation of drug targets and clinical development.

While the roles of MNKs in tumour development and progression have been well established, specific mono or dual MNK inhibitors with satisfactory levels of selectivity are still under development. Inhibitors like Cercosporamide [Cancer Biol Ther. 2015;16(5):648-656] or CGP57380 [Cancer Res. 2011;71:1849-1857], used for many years for the study of these kinases, are noted for their low selectivity [Biochem. J. 2007;408(3):297-315; Curr Med Chem. 2017;24(28):3025-3053].

MNKs belong to the serine/threonine-protein kinase family and are classified as members of the Ca²⁺/calmodulin-dependent kinases.

MNK kinases are present in two isoforms: MNK1 and MNK2. MNK1 is an inducible isoform, easily phosphorylated by ERK and p-38, whereas MNK2 has high basal activity.

While the overall structural architecture thereof resembles other protein kinases, MNKs contain several unusual elements [Oncotarget. 2012;3:118-131; Chem Biol. 2014;4:441-452]. These differences open up the possibility of developing highly selective inhibitors. Structural studies carried out in recent years have proposed different types of interactions between the ligands and active kinase sites: Type I (active kinase conformation), Type II (inactive kinase conformation), Type III (allosteric inhibitors) and Type VI (irreversible inhibitors) [Curr Med Chem. 2017;24(28):3025-3053].

Recently, the first two MNK inhibitors developed by Effector Therapeutics and Bayer AG (eFT508 and BAY 1143269, respectively) have entered clinical trials in oncology [J Med Chem. 2018;61(8):3516-3540; Cancer Lett. 2017;390:21-29]. According to the classification of MNK inhibitors, both are Type I inhibitors and act competitively for ATP. eFT508 has been successfully applied in the treatment combined with an mTOR inhibitor (everolimus) in large B-cell lymphomas, and BAY1143269 has been combined with taxanes in the treatment of non-small cell lung cancer. While these results confirm that MNK1/2 are a valuable target for preventing adaptation to approved treatments, the ATP-competitive mode of action may hinder wider use of these inhibitors in other types of cancers. In this regard, no results regarding the treatment of TNBC with both Type I inhibitors have been published.

In recent years, various research groups have been involved in the development of MNK1/2 inhibitors using various heterocyclic systems as a base structure. In particular, relevant to this patent application are those works that have used 1*H*-pyrazolo[3,4-*b*]pyridin-3-amine heterocyclic systems as the base structure for developing kinase inhibitors. Thus, US 2010/0113415 describes the use of said heterocycles as EphA4 receptor tyrosine kinase inhibitors which are useful in the treatment of neurological and neurodegenerative disorders, cancer and other conditions regulated by EphA4 receptor tyrosine kinase signalling. The two tested compounds in US 2010/0113415, labelled as Examples 63 and 67 provided a demosntrated effect as EphA4 receptor tyrosine kinase inhibitors. These assays do not illustrate or confirm, however, that they are useful in the treatment of cancers of hormone-dependent organs, such as breast cancer (including TNBC), ovarian cancer, endometrial cancer and prostate cancer, much less than all other listed compounds imply the said inhibitory effect of EphA4 receptor tyrosine kinase.

WO 2016/172010 A1 discloses the combination of a MNK inhibitor with an inhibitor, such as an antibody or si RNA, of PD-1 or PD-L1, and optionally with a chemotherapeutic agent, such as docetaxel, for use in the treatment of several cancers.

In Liu S. et al., "Inhibiting ERK/Mnk/elF4E broadly sensitizes ovarian cancer response to chemotherapy", Clinical And Translational Oncology, vol. 20, no. 3, pages 374 - 381 it is disclosed that ERK/Mnk/elF4E activation is critically involved in ovarian cancer chemoresistance and inhibiting ERK/Mnk/elF4E broadly sensitizes ovarian cancer response to chemotherapy.

In Vidya P. Ramamurthy et al., "Simultaneous targeting of androgen receptor (AR) and MAPK-interacting kinases (MNKs) by novel retinamides inhibits growth of human prostate cancer cell lines", Oncotarget, vol. 6, no. 5, pages 3195 - 3210 it is disclosed that simultaneous inhibition of AR and eIF4E activation is a novel and efficacious therapeutic approach for prostate cancer, and that Novel Retinamides hold significant promise for treatment of advanced prostate cancer.

In Choi CH et al, "Direct inhibition of elF4E reduced cell growth in endometrial adenocarcinoma". J Cancer Res Clin Oncol. 2011, vol. 137, no. 3, pages 463-469 it is disclosed that elF4E might be a promising therapeutic target in endometrial cancer.

In Hou S, et al. "Significance of MNK1 in prognostic prediction and chemotherapy development of epithelial ovarian cancer". Clin Transl Oncol. 2017, vol. 19, no. 9, pages 1107-1116 it is disclosed that MNK1 can act as a potential target for novel chemotherapy development towards epithelial ovarian cancer.

Furthermore, WO 2011/019780 claims the use of these heterocyclic systems as inhibitors of protein kinase enzymes for the treatment of allergic disorders, autoimmune and/or inflammatory diseases, and cancers. None of these patents, however, mention MNK1/2 or elF4E phosphorylation.

Lastly, WO 2015/004024 describes 1*H*-pyrazolo[3,4-*b*]pyridin-3-amine systems with a very bulky heterocyclic substituent at position C5 of the pyrazolopyridine system. In this case, the resulting systems are indeed claimed as inhibitors of MNK1 kinase and/or MNK2 kinase, mentioning their connection with eIF4E and their involvement in the treatment of breast cancer.

Thus, although many efforts have been done for providing effective cancer therapies, there is still a need of alternative ones and particularly for those cancer types that, in some way, become resistant or refractive to common therapies.

### Summary of Invention

Present invention results from the surprising MNK1/2 inhibitory effect of one of the simplest 1*H*-pyrazolo[3,4-*b*]pyridin-3-amine derivatives: compound 4,6-diphenyl-1H-pyrazolo[3,4-b]pyridin-3-amine, (named in this description as EB1).

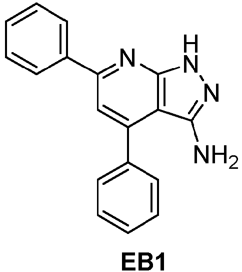

Said compound was first described in DE 2160780 (1973) as an intermediate for the synthesis of dyes and in other articles and patents as a synthesis intermediate (such as for example in US 2010/0113415). However, the possible biological activity thereof in connection with MNK1/2 has apparently not been described yet.

Inventors surprisingly found that EB1 could selectivity inhibit MNK1/2 (enzymatic IC₅₀ of 0.7 µM and an *in vitro* ECso of approximately 1.5 µM in triple-negative breast cancer cells (MDA-MB-231)). This inhibition was, in addition, a non-ATP competitive MNK1/2 inhibition, a genuine feature in relation with the other MNK1/2 inhibitors known and commercially available nowadays. Moreover, as MNK1/2 inhibitor it was no toxic when tested on normal cells.

Selectivity of EB1 was tested in a panel of 320 kinases which included EphA4 receptor tyrosine kinase. Although EB1 is mentioned as intermediate for the synthesis of other active compounds with inhibitory effect of this receptor in US 2010/0113415, no activity for EB1 could be inferred from the assays disclosed in this prior art document. Indeed, in an inhibitory test of EphA4 carried out with EB1 in this kinase panel using the same protocol, only an inhibition of 10% was obtained at 1 uM while the inhibition of MNK1 was 48%, so it is clear the virtual lack of inhibitory activity of EB1 against EphA4.

Thus, in a first aspect, the invention relates to 4,6-diphenyl-1*H*-pyrazolo[3,4-b]pyridin-3-amine or a pharmaceutically or veterinary acceptable salt thereof, for use in the treatment of a cancer of an hormone-dependent organ selected from breast cancer, prostate cancer, ovarian cancer and endometrial cancer.

It also forms part of the disclosure (but not of the claimed invention) the use of a therapeutically effective amount of EB1, or a pharmaceutically or veterinary acceptable salt thereof, for the preparation of a medicament for the treatment and/or prevention of a cancer of an hormone-dependent organ selected from breast cancer, prostate cancer, ovarian cancer and endometrial cancer.

Results from other derivatives studied with various substituents included in the phenyl rings at positions C4 and C6 of the 1*H*-pyrazolo[3,4-*b*]pyridin-3-amine system have presented activities lower than those of EB1. As will be illustrated in more detail in the description below, EB1 inhibits elF4E phosphorylation with *in vitro* EC₅₀ ~ 1.5 µM with almost complete inhibition as of 2.5 µM and the effect is fast and lasts for at least 72 h; the inhibitory effect seems to be due to the direct inhibition of MNKs; and noteworthy, EB1 shows excellent selectivity for MNK1/2 in relation with other kinases.

Inventors have also surprisingly found that when EB1 is used in combination with certain chemotherapeutic and/or immunotherapeutic compounds or agents of those commonly used in the treatment of these cancers of hormone-dependent organs, the observed therapeutic effect is synergistically increased in terms of one or more of reduced cell growth and induction of apoptosis.

Thus, a second aspect of the invention relates to combinations comprising:
a) a therapeutically effective amount of EB1 or a pharmaceutically or veterinary acceptable salt thereof; and
b) a therapeutically effective amount of one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof; wherein the combination is for use in the treatment of a cancer of an hormone-dependent organ selected from breast cancer, prostate cancer, ovarian cancer and endometrial cancer.

These combinations including EB1 are proposed, without being bound to therory, because EB1 as MNK inhibitor makes possible to sensitise tumour cells to common therapies, to which common therapies certain subjects or population have developed resistance. Resistance to a cancer therapy is in part developed, as indicated above, due to the deregulation of protein synthesis in response to cell stress caused by toxic agents,such as a compound for treating cancer as a cell protection mechanism.The MNKs are required under cellular stress (activated by the stress pathways), which often prevents classical therapies to be effective. Targeting MNKs with EB1 or a salt thereof sensitizes the cells to standard of care treatments that fail due to the activation of stress pathways.

The compounds in combinations of the invention may be formulated in different types of compositions/kits of parts. Thus, a third aspect of the invention relates to a single pharmaceutical or veterinary composition which comprises:
a) a therapeutically effective amount of EB1 or a pharmaceutically acceptable salt thereof; and
b) a therapeutically effective amount of one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof, together with one or more pharmaceutically or veterinary acceptable excipients or carriers;
wherein the single pharmaceutical or veterinary composition is for use in the treatment of a cancer of an hormone-dependent organ selected from breast cancer, prostate cancer, ovarian cancer and endometrial cancer.

A fourth aspect aspect of the invention relates to a package or kit of parts comprising:
i) a first pharmaceutical or veterinary composition which comprises a therapeutically effective amount of EB1, or a pharmaceutically or veterinary acceptable salt thereof, together with one or more pharmaceutically or veterinary acceptable excipients or carriers;
ii) a second pharmaceutical or veterinary composition which comprises a therapeutically effective amount of one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof, together with one or more pharmaceutically or veterinary acceptable excipients or carriers;
iii) instructions for the use in combination of i) and ii);

wherein the first and second compositions are separate compositions;
wherein the package or kit of parts is for use in the treatment of a cancer of an hormone-dependent organ selected from breast cancer, prostate cancer, ovarian cancer and endometrial cancer.

Finally, precisely due to the selectivity for MNK1/2, it is also herewith disclosed (but not claimed) the non-therapeutically use of 4,6-diphenyl-1*H*-pyrazolo[3,4-*b*]pyridin-3-amine, or of a pharmaceutically or veterinary acceptable salt thereof, as a selective, non-cytotoxic inhibitor of MAP kinase-interacting serine/threonine-protein kinase 1 (MNK1) and MAP kinase-interacting serine/threonine-protein kinase 2 (MNK2). This use is of interest as inhibitor of MNK1/2 in an isolated sample comprising mammal cells (i.e. isolated biopsies of tumour tissue, stablished cell lines, etc.) with the aim testing therapeutic combinations, or as reagent in biochemistry assays in which isolated mammal cells are used to study mechanistic cell processes, metabolic pathways or to test substances in screening methods. The inhibition of MNK1 and MNK2 by means of EB1 causes the inhibition of elF4E phosphorylation.

### Brief Description of Drawings

To complement the description that is being made and for the purpose of helping to better understand the features of the invention, a set of drawings is attached as an integral part of said description, wherein the following has been depicted, with an illustrative and non-limiting character:
Figure 1 shows an example of the titration curves of EB1 in MDA-MB-231 cells which show no effect on upstream proteins and which demonstrate selective MNK inhibition. The results were equivalent at 24 h and 48 h. Final concentration of dimethyl sulfoxide (DMSO) 0.5 %. CGP57380 (CGP) is used as a positive control and DMSO as a negative control.
Figure 2 shows titration curve EB1 in MCF10 cells. Final concentration of DMSO 0.5 %. CGP57380 (CGP) is used as a positive control and DMSO as a negative control.
Figure 3 shows proliferation curves for EB1 in MDA-MB-231 and MCF10 cells. CGP57380 (CGP) and EFT508 are used as positive controls and DMSO as a negative control. Norm. Abs. is the normalized absorbance and T(h) is the time in hours.
Figure 4 shows the MDA-MB-231 cell cycle treated with EB1. 100 nM doxorubicin is used as a positive control and DMSO as a negative control.
Figure 5 shows a kinome for EB1.
Figure 6 shows a titration curve of EB1 in A375M, MV4-11 and MDA-MB-468 cells. Final concentration of DMSO 0.5 %. CGP57380 (CGP) is used as a positive control and DMSO as a negative control.
Figure 7 shows that the combination of doxorubicin (DOXO) with EB1 increases the efficacy of the chemotherapeutic drug (B) and reverses the increase in p-eIF4E caused by cellular stress related to treatment with doxorubicin (A).
Figure 8 shows that treatment of IMR90 cells, non-transformed fibroblasts, does not cause significant cell growth arrest (Figure A, where RCG is relative cell growth ) and does not induce cell death (Figure B, % Apopototic cells). In contrast, in all tested breast cancer cell lines (MDA-MB231, MCF7 and MDA-MB468) cell growth is inhibited in a dose dependent manner. DMSO means dimethylsulfoxide; CDDP is cisplatin; RCG is relative cell growth
Figure 9 shows tha EB1 treatment of MCF7 cells results in increased sensitivity to tamoxifen. CGP means CGP57380.
Figure 10 shows thatEB1 treatment reduces levels of PD-L1 in the human breast cancer cell line MDA-MB231. At comparable inhibition rates of elF4E phosphorylation, EB1 is equally potent or superior to the MNK inhibitor eFT508 and CGP57380.

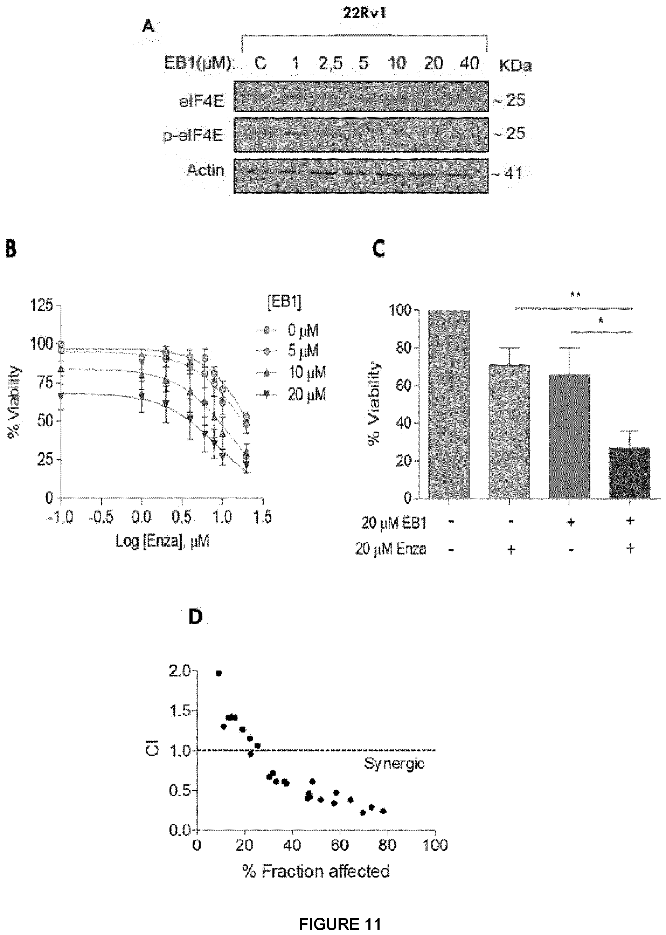

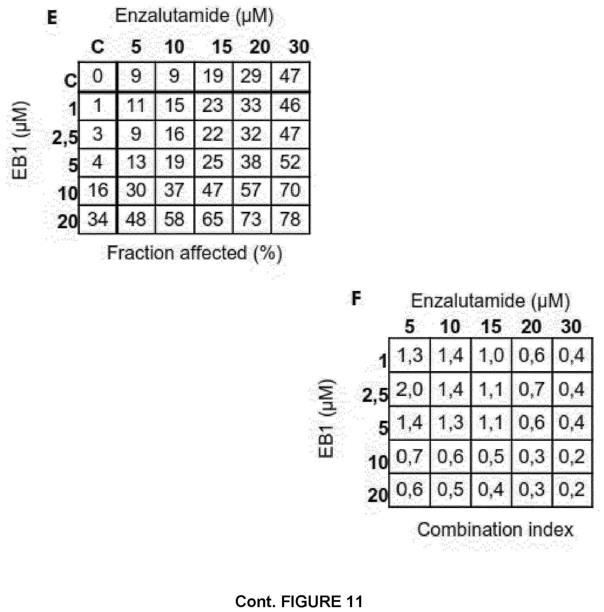

Figure 11 shows cell viability under combined inhibition of AR and elF4E phosphorylation. (A) Dose dependent inhibition of elF4E phosphorylation after 24h EB1 treatment in 22Rv1. Synergistic effect of the EB1 and Enzalutamide with 22Rv1 (B-F) cell viability assays. (B) Number of viable cells after 72h treatment with pairwise combinations reported as percentage relative to control. (E) Cell viability data presented as a grid displaying the percentage of affected cells by each pairwise combination of drug doses. (C) Drug combinations with the strongest inhibition in cell viability (bars; mean ± SEM; n=3. *p<0.05, **p<0.01 two-tailed Student's test). (F) Combination index (CI) presented as a grid for each pairwise combination of drug doses calculated by the Chou-Talalay method at non-constant ratio. (D) Fraction affected vs. combination index (CI) plots.

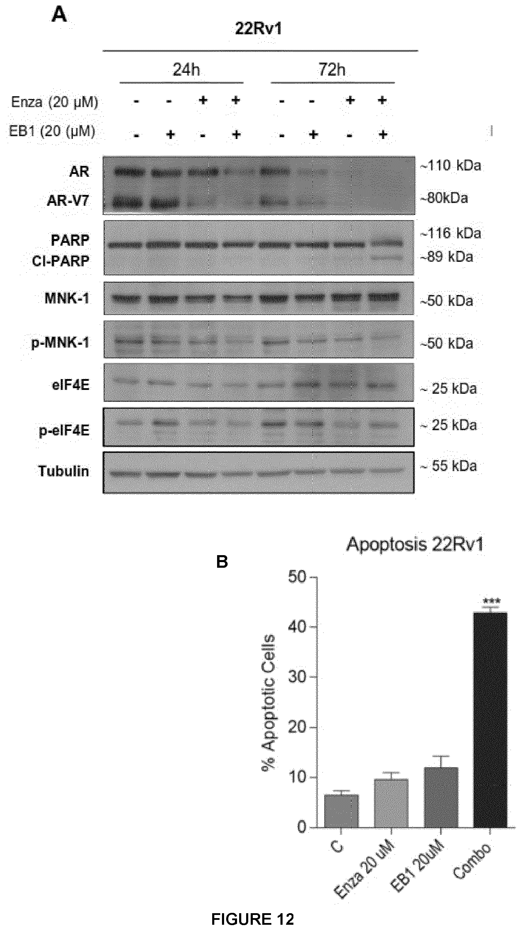

Figure 12 shows that dual inhibition of AR and elF4E phosphorylation induces cell death via apoptosis in cell lines 22Rv1 (A) cells were treated either with vehicle (DMSO), enzalutamide, EB1 or a combination (Combo) of both with the indicated concentrations for 72h. (B) Apoptosis assays of 22Rv1 cell line after treatment with the indicated concentrations of Enzalutamide, EB1 and the combination of both for 72h, quantified by analysis of fragmented/condensed chromatin after Propidium Iodide staining. Data are mean of two technical replicates from one experiment ±SEM ***p<0.001 two-tailed Student's test

### Detailed description of the invention

Unless otherwise stated, all terms as used in this application shall be understood in their ordinary meaning as known in the art. Other more specific definitions for certain terms as used in the present application are as set forth below and are intended to apply uniformly throughout the specification and claims unless an otherwise expressly set out definition provides a broader meaning.

As used herein, the indefinite articles "a" and "an" are synonymous with "at least one" or "one or more." Unless indicated otherwise, definite articles used herein, such as "the," also include the plural of the noun.

The term "cancer of hormone-dependent organs" includes cancers of organs that are dependent of hormones for their function, in particular of sex hormones (androgens, estrogens, progestogens) for their function or even that secrete such sex hormones, as reproductive and sexual organs are, including breast, ovarian, endometrium, prostate and testicles. Within these cancers of hormone-dependent organs there are included: (a) those hormone-dependent cancers, thus cancers that are dependent on a hormone for growth and/or survival, also called "hormone-sensitive"; (b) cancers that become independent of hormones during the disease progression; and (c) hormone-independent cancers but with origin in hormone-dependent organs, such as TNBC.

PD-1/PD-L1 stands for "inhibitors of the programmed cell-death protein 1 and its ligand. The section of examples includes assays to determine the inhibitory effect, and the skilled person in the art knows also how to test this inhibitory effect.

The expression "therapeutically effective" as used herein throughout this description, refers to the amount of a compound or combination of compounds that, when administered, is enough to prevent development of, or alleviate to some extent, one or more of the symptoms of the disease which is addressed. In this particular description, it is the amount of a compound, combination of compounds, or composition that produces a desired therapeutic effect in a subject, such as treating cancer, in particular cancer of hormone.dependent organs, including breast cancer and prostate cancer. The precise therapeutically effective amount is an amount of the composition that will yield the most effective results in terms of therapeutic efficacy in a given subject. The specific dose of the EB1 to obtain a therapeutic benefit may vary depending on the particular circumstances of the individual patient including, among others, the size, weight, age and sex of the patient, the nature and stage of the disease, the aggressiveness of the disease, and the route of administration. The specific dose of EB1 to obtain a therapeutic benefit when administered in the herewith disclosed combinations, compositions or kit of parts, may vary in relation with the specific dose of the compound used as single active agent.

There is no limitation on the type of salt of the compounds EB1 that can be used, provided that these are pharmaceutically or veterinary acceptable when they are used for the therapeutic purpose. The term "pharmaceutically or veterinary acceptable salts", embraces salts commonly used to form alkali metal salts and to form addition salts of free acids or free bases. The preparation of pharmaceutically or veterinary acceptable salts of the compounds EB1 can be carried out by methods known in the art.

For instance, they can be prepared from the parent compound,by conventional chemical methods. Generally, such salts are, for example, prepared by reacting the parent compound EB1 with a stoichiometric amount of the appropriate pharmaceutically or veterinary acceptable base or acid in water or in an organic solvent or in a mixture of them. The compound EB1 and their respective salts may differ in some physical properties but they are equivalent for the purposes of the present invention.

The compound EB1 may be as an amorphous or crystalline solid form either as free solvation compound or as solvates (e.g. hydrates) and it is intended that all forms are within the scope of the present invention for the purposed therapeutic use and for the disclosed combinations. Methods of solvation are generally known within the art. In general, the solvated forms with pharmaceutically, or veterinary acceptable solvents such as water, ethanol and the like are equivalent to the unsolvated form for the purposes of the invention. In particular, the compound EB1 is used according to this description as an amorphous solid.

In all embodiments of the invention referring to the EB1, the pharmaceutically or veterinary acceptable salts thereof are always contemplated even if they are not specifically mentioned.

The expression "pharmaceutically or veterinary acceptable excipients or carriers" refers to pharmaceutically or veterinary acceptable materials, compositions or vehicles. Each component must be pharmaceutically or veterinary acceptable in the sense of being compatible with the other ingredients of the pharmaceutical or veterinary composition. It must also be suitable for use in contact with the tissue or organ of humans and animals without excessive toxicity, irritation, allergic response, immunogenicity or other problems or complications commensurate with a reasonable benefit/risk ratio.

The phrases "patient" and "subject" are used interchangeably herein, and they relate to any mammal, in particular they relate to human.

When in this description the expression "treatment of cancer" is used it relates to any of the treatment and/or the prevention of the indicated cancer, although not specifically stated.

As previously indicated, a first aspect of the invention relates to 4,6-diphenyl-1*H-*pyrazolo[3,4-b]pyridin-3-amine (EB1) or a pharmaceutically acceptable salt thereof, for use in the treatment of a cancer of an hormone-dependent organ selected from breast cancer, prostate cancer, ovarian cancer and endometrial cancer.

All these cancers in which 4,6-diphenyl-1/7-pyrazolo[3,4-b]pyridin-3-amine or a pharmaceutically acceptable salt thereof is proposed, are cancers also referable by inhibition of MNK1 and MNK2, in which over-expression of p-eIF4E takes place. Indeed, other cancers in which such p-eIF4E overexpression takes place, and also disclosed (but not claimed) herewith are selected from the group consisting of lung cancer, colon cancer, melanoma, sarcoma, leukaemia, lymphomas and brain (i.e. glioma) tumours. In all these cancers that can be treated with EB1 or a pharmaceutically or veterinary acceptable salt thereof, pelF4E overexpression is induced by treatments selected from the group consisting of chemotherapy, radiotherapy and immunotherapy.

Within the cancers of an hormone-dependent organs, the hormone-dependent cancers are commonly treated mainly by blocking interaction of the hormones with the corresponding cell receptors in tumour cells. This makes that stress pathway activates and leads cancer cells to evade the treatment (resistance to treatment), because said tumour cells become no sensitive. These resistant cancers are those that have become independent of hormones during the disease progression and their treatment is challenging. Finally, the hormone-independent cancers that origin in hormone-dependent organs, such as TNBC, are cancers that as such do not respond to hormone or antihormone therapy, because they lack hormone receptors or express receptors that lack the hormone or antihormone binding domain.

Thus, in a particular embodiment of the first aspect, EB1 or a pharmaceutically or veterinary salt thereof, is for use in the treatment of a cancer of an hormone-dependent organ selected from breast cancer, prostate cancer, ovarian cancer and endometrial cancer in a population of subjects that have developed resistance, or that are as such resistant, to targeted chemotherapy and/or resistant to immunotherapy commonly used for these cancers.

For "targeted chemotherapy" is to be understood that the cancer is treated using drugs to target specific genes and proteins that are involved in the growth and survival of cancer cells. Targeted therapy can affect the tissue environment that helps a cancer grow and survive or it can target cells related to cancer growth, like blood vessel cells.

This is noteworthy in the case of TNBC, as mentioned,characterised by the absence ER, PR and HER-2, and thus only cured by means of surgery, radiotherapy and/or systemic chemotherapy. Assurance that the systemic chemotherapy in TNBC is effective, or resistance is not developed, can be attained (see examples below) if EB1 or a pharmaceutically or veterinary salt thereof is used in this particular breast cancer.

Most breast carcinomas are estrogen positive an have better prognosis but high risk of late recurrences. In fact, patients are treated with antiestrogens for many years. And new pharmacological approaches are being studfied , including the combination with mnk inhibitors
Also noteworthy is for prostate cancer, specially in the context of castration resistance, where the tumor cells are no longer controlled by androgen suppresion but the AR remains playing a critical role, in many cases due to the acquisition of alterations such as amplifications, activating mutations on the ligand binding domain (LBD) and splice variants, particularly the consitutively active variants that lack the LBD (i.e. AR-V7). Such AR modifications allow its activation in the absence of hormones and the development of clinical resistance to potent AR-targeted therapies (i.e. enzalutamide, AR-inhibitor that binds to the AR-LBD).

Therefore, EB1 or its salts are proposed for use in the treatment of a cancer of an hormone-dependent organ selected from breast cancer, prostate cancer, ovarian cancer and endometrial cancer in patients non-sensitive to common therapies and/or in patients that, after the treatment with the commonly used therapies, develop resistance due to a cell phenotype change (deregulation of protein synthesis in response to cell stress). Advantadgeously, this approach allows to re-sensitize those patients that have evolved to a non-respondent profile, and make the common targeted therapy effective again.

Thus, in another particular embodiment of the first aspect, EB1 is for use in the treatment of a cancer of an hormone-dependent organ selected from breast cancer and prostate cancer.

In another more particular embodiment, optionally in combination with any embodiment above or below, the breast cancer is the triple negative breast cancer (TNBC).

Also in another more particular embodiment, optionally in combination with any embodiment above or below, the cancer of an hormone-dependent organ is prostate cancer.

In yet another particular embodiment of the first aspect, the 4,6-diphenyl-1*H*-pyrazolo[3,4-*b*]pyridin-3-amine (EB1) or a pharmaceutically acceptable salt thereof for use as indicated above, is such that the treatment comprises administering 4,6-diphenyl-1*H*-pyrazolo[3,4-*b*]pyridin-3-amine (EB1) or a pharmaceutically or veterinary acceptable salt thereof, in combination with one or more compounds selected from compounds for chemotherapy and compounds for immunotherapy.

In a more particular embodiment when EB1 or a salt thereof is for the mentioned use in combination with other compounds for chemotherapy or immunotherapy, and optionally in combination with one or more features of the various embodiments described above or below throughout all the description, the treatment comprises the simultaneous, concurrent, separate or sequential administration of: (a) EB1, or a pharmaceutically or veterinary acceptable salt thereof; and (b) compounds selected from compounds for chemotherapy and compounds for immunotherapy.

In another particular embodiment of the first aspect, the chemotherapeutic compounds are selected from doxorubicin, tamoxifen, enzalutamide, docetaxel, cisplatin, lapatinib and combinations thereof.

In yet another more particular embodiment EB1, or a pharmaceutically or veterinary acceptable salt thereof, is for use as disclosed in the first aspect in combination with doxorubicin.

In yet another more particular embodiment EB1, or a pharmaceutically or veterinary acceptable salt thereof, is for use as disclosed in the first aspect in combination with tamoxifen.

In yet another more particular embodiment EB1, or a pharmaceutically or veterinary acceptable salt thereof, is for use as disclosed in the first aspect in combination with enzalutamide.

In yet another more particular embodiment EB1, or a pharmaceutically or veterinary acceptable salt thereof, is for use as disclosed in the first aspect in combination with docetaxel.

Also in another particular embodiment of the first aspect, optionally in combination with any embodiment above or below, the compounds for immunotherapy comprise inhibitors of the programmed cell-death protein 1 and its ligand (PD-1/PD-L1).

Examples of PD1 inhibitors include the approved Pembrolizumab, Nivolumab, Cemiplimab. Other examples still in experimental phase are Partalizumab, Camrelizumab (SHR1210), Sintilimab (IBI308), Tislelizumab (BGB-A317), Toripalimab (JS 001), Dostarlimab (TSR-042, WBP-285), INCMGA00012 (MGA012), AMP-224 and AMP-514 (MEDI0680).

Examples of PD-L1 inhibitors include the approved Atezolizumab, Avelumab, Durvalumab. Other examples still in experimental phase are KN035, CK-301, AUNP12, CA-170, and BMS-986189.

Present invention relates in a second aspect to particular new combinations of chemotherapeutic or immunotherapeutic compounds with EB1, or a pharmaceutically or veterinary acceptable salt thereof, such as the combinations comprising:
a) a therapeutically effective amount of EB1 or a pharmaceutically or veterinary acceptable salt thereof; and
b) a therapeutically effective amount of one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof;
wherein the combination is for use in the treatment of a cancer of an hormone-dependent organ selected from breast cancer, prostate cancer, ovarian cancer and endometrial cancer.

In a particular embodiment of this second aspect, the combination for use comprises a therapeutically effective amount of EB1, or a pharmaceutically or veterinary acceptable salt thereof, and a therapeutically effective amount of one or more of enzalutamide and docetaxel. These combinations are in particular for use in the treatment of prostate cancer.

In also another particular embodiment of the second aspect, the combination for use comprises a therapeutically effective amount of EB1, or a pharmaceutically or veterinary acceptable salt thereof, and a therapeutically effective amount of one or more of doxorubicin and tamoxifen. These combinations are in particular for use in the treatment of breast cancer, and more in particular the triple negative breast cancer.

In also another particular embodiment of the second aspect, the combination for use comprises a therapeutically effective amount of EB1, or a pharmaceutically or veterinary acceptable salt thereof, and a therapeutically effective amount of one or more of an inhibitor of PD-1 or PD-L1.

Another aspect of the invention is a single pharmaceutical or veterinary composition which comprises:
a) a therapeutically effective amount of EB1, or a pharmaceutically or veterinary acceptable salt thereof; and
b) a therapeutically effective amount of one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof, together with one or more pharmaceutically or veterinary acceptable excipients or carriers;
wherein the single pharmaceutical or veterinary composition is for use in the treatment of a cancer of an hormone-dependent organ selected from breast cancer, prostate cancer, ovarian cancer and endometrial cancer.

In a particular embodiment of this single pharmaceutical or veterinary composition for use, it comprises a therapeutically effective amount of EB1, or a pharmaceutically or veterinary acceptable salt thereof, and a therapeutically effective amount of doxorubicin.

In another particular embodiment of the single pharmaceutical or veterinary composition for use, it comprises a therapeutically effective amount of EB1, or a pharmaceutically or veterinary acceptable salt thereof, and a therapeutically effective amount of tamoxifen.

In another particular embodiment of the single pharmaceutical or veterinary composition for use, it comprises a therapeutically effective amount of EB1, or a pharmaceutically or veterinary acceptable salt thereof, and a therapeutically effective amount of enzalutamide.

In another particular embodiment of the single pharmaceutical or veterinary composition for use, it comprises a therapeutically effective amount of EB1, or a pharmaceutically or veterinary acceptable salt thereof, and a therapeutically effective amount of docetaxel.

In another particular embodiment of the single pharmaceutical or veterinary composition for use, it comprises a therapeutically effective amount of EB1, or a pharmaceutically or veterinary acceptable salt thereof, and a therapeutically effective amount of an inhibitor of the programmed cell-death protein 1

In another particular embodiment of the single pharmaceutical or veterinary composition for use, it comprises a therapeutically effective amount of EB1, or a pharmaceutically or veterinary acceptable salt thereof, and a therapeutically effective amount of an inhibitor of the ligand of the programmed cell-death protein 1.

In another particular embodiment, optionally in combination with one or more features of the various embodiments described above or below throughout all the description, the invention also relates to a package or kit of parts comprising:
i) a first pharmaceutical or veterinary composition which comprises a therapeutically effective amount of EB1, or a pharmaceutically or veterinary acceptable salt thereof, together with one or more pharmaceutically or veterinary acceptable excipients or carriers;
ii) a second pharmaceutical or veterinary composition which comprises a therapeutically effective amount of one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof, together with one or more pharmaceutically or veterinary acceptable excipients or carriers;
iii) instructions for the use in combination of i) and ii);
wherein the first and second compositions are separate compositionswherein the package or kit of parts is for use in the treatment of a cancer of an hormone-dependent organ selected from breast cancer, prostate cancer, ovarian cancer and endometrial cancer.

The selection of the pharmaceutical or veterinary formulation will depend upon the nature of the active compound and its route of administration. Any route of administration may be used, for example oral, parenteral and topical administration.

For example, the pharmaceutical or veterinary composition may be formulated for oral administration and may contain one or more physiologically compatible carriers or excipients, in solid or liquid form. These preparations may contain conventional ingredients such as binding agents, fillers, lubricants, and acceptable wetting agents. The pharmaceutical or veterinary composition may be formulated for parenteral administration in combination with conventional injectable liquid carriers, such as water or suitable alcohols. Conventional pharmaceutical or veterinary excipients for injection, such as stabilizing agents, solubilizing agents, and buffers, may be included in such compositions. These pharmaceutical or veterinary compositions may be injected intramuscularly, intraperitoneally, or intravenously.

The pharmaceutical composition may be formulated for topical administration.

Formulations include creams, lotions, gels, powders, solutions and patches wherein the compound is dispersed or dissolved in suitable excipients.

The pharmaceutical compositions may be in any form, including, among others, tablets, pellets, capsules, aqueous or oily solutions, suspensions, emulsions, or dry powdered forms suitable for reconstitution with water or other suitable liquid medium before use, for immediate or retarded release.

The appropriate excipients and/or carriers, and their amounts, can readily be determined by those skilled in the art according to the type of formulation being prepared.

It forms part of the invention the combination, single pharmaceutical or veterinary composition, package or kit of parts comprising a therapeutically effective amount of EB1, or a pharmaceutically or veterinary salt thereof; and one or more of the chemotherapeutic or immunotherapeutic compounds as listed above, for use in the treatment and/or prevention of cancer as defined in the claims in a subject in need thereof. In an aspect of the disclosure, they can be for use in cancers selected from the group consisting of breast cancer, lung cancer, ovarian cancer, endometrial cancer, colon cancer, prostate cancer, melanoma, sarcoma, leukaemia, lymphomas and brain tumours, including glioma. According to the invention, they are for use in the treatment of cancers of hormone-dependent organs as defined in the claims in a subject in need thereof. More in particular, for use in breast and prostate cancer in a subject in need thereof.

Thus, any of the combinations selected from the following group are, in particular, for use in the treatment of a cancers of an hormone-dependent organ selected from breast cancer, prostate cancer, ovarian cancer and endometrial cancer:
- the combination that comprises a therapeutically effective amount of EB1 and a therapeutically effective amount of one or more of enzalutamide and docetaxel;
- the combination that comprises a therapeutically effective amount of EB1 and a therapeutically effective amount of one or more of doxorubicin and tamoxifen; and
- the combination that comprises a therapeutically effective amount of EB1 and a therapeutically effective amount of one or more of an inhibitor of PD-1 or PD-L1.

It also forms part of the disclosure (but not of the claimed invention) the use of a combination comprising a therapeutically effective amount of EB1, or a pharmaceutically or veterinary acceptable salt thereof; and one or more of the chemotherapeutic or immunotherapeutic compounds selected from the group consisting of doxorubicin, tamoxifen, enzalutamide, docetaxel, cisplatin, lapatinib , inhibitors of the programmed cell-death protein 1 and its ligand (PD-1/PD-L1), and combinations thereof, together with one or more pharmaceutically or veterinary acceptable excipients or carriers; for the preparation of a medicament for the treatment and/or prevention of a cancer of an hormone-dependent organ selected from breast cancer, prostate cancer, ovarian cancer and endometrial cancer. More in particular, for the treatment and/or prevention of breast and prostate cancer.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. The invention is as defined in the claims.

### Examples

### Example 1. Synthesis of 4,6-diphenyl-1H-pyrazolo[3,4-b]pyridin-3-amine (EB1)

4,6-diphenyl-2-methoxynicotinonitrile (Journal of Heterocyclic Chemistry, 26(6), 1665-73; 1989) (0.6 mmol) is dissolved in 4 ml of 1,4-dioxane together with POBr₃ (1.34 mmol), pyridine-HBr (0.015 mmol) and H₃PO₄ (0.026 mmol). The mixture is heated under reflux for 18 hours at 120 °C. The reaction is stopped by adding cold water and neutralised with NaOH (6M). The solid obtained is filtered and washed with cold water to obtain 4,6-diphenyl-2-bromonicotinonitrile in a yield of 84 % and as a white solid, m. p.: 123-125 °C; ¹H NMR (400 MHz, DMSO-*d*₆) δ (ppm): 8.26 (s, 1H), 8.26-8.22 (m, 2H), 7.82-7.77 (m, 2H), 7.63-7.60 (m, 3H) and 7.59-7.55 (m, 3H); ¹³C NMR (100 MHz, DMSO-*d*₆) δ (ppm): 159.3, 156.2, 144.4, 135.5, 135.3, 131.3, 130.5, 129.1, 128.9, 127.7, 119.7, 116.5, 109.7; IR (KBr) vmax (cm⁻¹): 3432, 3029, 2225, 1649, 1575, 1517, 1493, 1419, 1373, 772, 747, 702, 686; MS (70 eV, El) m/z (%): 336.1 (96 %), 335.1 (68 %), 334.1 (100 %), 333.1 (52 %), 286.2 (36 %), 285.2 (46 %), 255.1 (69 %), 254.1 (20 %), 253.1 (31 %), 228.1 (26 %), 227.1 (55 %), 100.1 (20 %), 77.1 (43 %), 51.0 (22 %); Elemental analysis: calculated for C₁₈H₁₁BrN₂: C: 64.50 %, H: 3.31 %, N: 8.36 %; obtained: C: 64.83 %, H: 3.58 %, N: 8.31 %.

Next, 4,6-diphenyl-2-bromo-nicotinonitrile (0.18 mmol) and hydrazine monohydrate (0.36 mmol) are added to a 5 ml microwave vial and dissolved in 3 ml MeOH. The mixture is heated under microwave irradiation for 2 hours at 140 °C. The solvent is removed under reduced pressure. The solid obtained is resuspended in MeOH, filtered and washed with cold methanol to obtain 4,6-diphenyl-1*H*-pyrazolo[3,4-*b*]pyridin-3-amine which is purified by column chromatography (Cy: AcOEt gradient 0 % to 20 % in 10 minutes, 20 % isocratic for 5 minutes, 20 % to 100 % in 15 minutes). Yield 71 %, yellowish solid, m. p.: 219-220 °C; ¹H NMR (400 MHz, DMSO-*d*₆) δ (ppm): 12.37 (s, 1H), 8.21 - 8.15 (m, 2H), 7.73 - 7.67 (m, 2H), 7.63 - 7.47 (m, 7H), 4.56 (s, 2H); ¹³C NMR (100 MHz, DMSO-*d*₆) δ (ppm): 155.5, 153.3, 147.2, 145.4, 138.9, 137.2, 129.2, 128.9, 128.8, 128.8, 128.7, 127.2, 112.4, 102.0; IR (KBr) vmax (cm⁻¹): 3423, 3297, 3193, 1737, 1623, 1591, 1567, 1525, 1401, 1354, 1292, 702; MS (70 eV, El) m/z (%): 287.1 (21 %), 286.1 (100 %), 285.1 (38 %); Elemental analysis: calculated for C₁₈H₁₄N₄: C: 75.50 %, H: 4.90 %, N: 19.60 %; obtained: C: 75.43 %, H: 4.90 %, N: 19.56 %.

EB1 synthesized as illustrated in this Example 1 is the one used in the Examples 2-6 that follow.

### Example 2. EB1 inhibitory effect

### Materials and methods:

### Kinase activity assay:

The kinase activity of the compound was measured by a radiometric kinase assay (³³PanQinase^{®} Activity Assay) carried out by Proqinase (www.proqinase.com).

The assays were carried out in 96-well FlashPlates^{™} by PerkinElmer (Boston, MA, USA ) with a reaction volume of 50 µl. The reaction cocktail was pipetted in 4 steps: (1) 20 µl buffer, (2) 5 µl ATP solution (in H₂O), (3) 5 µl compound (in 10 % DMSO) and (4) 20 µl enzyme/substrate mixture. Lastly, the cocktail contained 70 mM HEPES-NaOH pH 7.5, 3 mM MgCl₂, 3 mM MnCl₂, 3 µM sodium orthovanadate, 1.2 mM DTT, 50 µg/ml PEG₂₀₀₀₀, ATP (1 µM for MNK1 and 0.3 µM for MNK2), [γ-³³P] -ATP (approx. 1.2 x 1006 cpm per well), protein kinase (variable amounts depending on the batch) and substrate (2 µg/50 µl).

Reaction cocktails were incubated at 30 °C for 60 minutes. The reaction was stopped with 50 µl of 2 % H₃PO₄ (v/v), the plates were sucked, they were washed twice with 200 µl of 0.9 % NaCl (w/v) and the incorporation of ³³Pi (MicroBeta microplate scintillation counter, Wallac) was determined. All assays were performed with a Beckman Coulter/SAGIAN^{™} system.

The residual activity (in %) for each well of a particular plate was calculated using the following formula (wherein high control is the kinase activity in absence of an inhibitor and low control is the radioactivity value of the substrate in absence of a kinase). Res. Activity (%) = 100 X [(signal of the compound - low control) / (high control - low control)]

To screen compounds, the residual activity of both kinases was analysed after treatment with 10 µM of the compounds.

To measure IC₅₀, residual kinase activity was analysed at 10 different concentrations in a range of 5·10⁻⁴ M to 1.5·10⁻⁹ M.

To study selectivity, residual activity was measured at 1 µM from a 320-kinase panel.

### Cell culture:

Comercially available MDA-MB-231, MDA-MB-468, MCF7 and A375M, IMR90 (IMR-90 (ATCC^{®} CCL-186^{™}) cells were grown at 37 °C and 5 % CO₂ in DMEM medium (Dulbecco's Modified Eagle Medium, 4.5 g/l glucose; 30 mg/l L-Glutamine (Gibco)) supplemented with 10 % foetal bovine serum, 100 U/ml penicillin and 100 µg/ml streptomycin.

MCF10A cells (also commercially available) were grown in DMEM/F-12 medium (Dulbecco's Modified Eagle Medium: 3.125 g/L D-Glucosa, 365 mg/L L-Glutamine, Nutrient Mixture F-12 (Gibco)) supplemented with 10% FBS, 100 U/ml penicillin, 100 µg/ml streptomycin, 1% L-Glutamine (Cambrex), 10 ng/ml choleric toxin (Sigma-Aldrich), 0.005 mg/ml insulin (Sigma), 100 ng/ml hydrocortisone (Sigma) and 20ng/ml EGF (Epidermal growth factor, Sigma).

MV4-11 cells were grown in IMDM medium (Iscove's Modified Dulbecco's Medium, Gibco) supplemented with 10% FBS, 1% L-glutamine, 100 U/ml penicillin and 100 µg/ml streptomycin.

### In vitro proliferation assays:

In 96-well plates, 5000 cells were seeded for each condition. After 24 h, the cells were treated with the compound at the corresponding concentration. The stocks of compounds were prepared in 100 % DMSO at a concentration 200 times more concentrated than the treatment concentration. Thus, in all cases, the final concentration of DMSO in the medium is 0.5 %. After incubation, the medium was removed and the cells were fixed with 4 % PFA (100 µl per well) for 30 minutes. Two washes with PBS were performed.

Cells were stained with crystal violet (0.5 % in EtOH): 100 µl were added to each well, stirred for 15 minutes, washed with water and left to dry. The content of each well was dissolved in 200 µl of 15 % AcOH and the absorbance thereof was measured at 595 nm.

*In vitro* assays for inhibition of elF4E phosphorylation:
Inhibition of elF4E phosphorylation was quantified by Western Blot. Cells were seeded in 60 mm plates (700,000 cells for 24 h, 500,000 cells for 48 h and 300,000 cells for 72 h). After 24 h, the treatments were added. The stocks of compounds were prepared in 100 % DMSO at a concentration 200 times more concentrated than the treatment concentration. Thus, in all cases, the final concentration of DMSO in the medium was 0.5 %.

After incubation, proteins were removed and Western Blot was performed. On ice, the plates were washed with PBS and the cells were lysed with 60 µl lysis buffer (50 mM Tris, 200 mM NaCl, 5 mM EDTA, 0.1 % Triton 100x, pH 7.5 supplemented with protease inhibitors (EDTA-free Protease Inhibitor Cocktail Set III, Calbiochem) and phosphatase inhibitors (Phosphatase Inhibitor Cocktail Set II, Calbiochem)). The samples were centrifuged at 15,000 g and the supernatant was quantified by the Bradford method. The samples were all diluted to the same concentration with a lysis buffer and loading buffer (Laemmli buffer) and denatured for 5 minutes at 95 °C. The proteins were separated on a 12 % agarose gel (20 µg were loaded per well) and transferred to a PDVF (Polyvinylidene fluoride) membrane. β-Actin antibodies, abbreviated as "actin" in the figures (Calbiochem), elF4E (anti-rabbit, Cell signalling), and phosphorylated-elF4E (S209) (anti-rabbit, Cell signalling) in 5 % BSA in TBST were used to study the activity of the compounds. Selectivity against MNK was analysed with ERK, p-ERK, MNK1, p-MNK1, 4EBP1 and p-4EBP1 antibodies.

### Results:

The present invention relates to *H*-pyrazolo[3,4-*b*]pyridin-3-amine EB1, orefa pharmaceutically acceptable salt thereof, as a selective MNK inhibitor for use in the treatment of triple-negative breast cancer and other referable cancers with p-eIF4E overexpression due to increased MNK activity as defined in the claims.

As previously announced, and surprisingly, EB1 has an enzymatic IC₅₀ of 0.7 µM and an *in vitro* ECso of approximately 1.5 µM in triple-negative breast cancer cells (MDA-MB-231). Complete inhibition of eIF4E phosphorylation is achieved without cytotoxic effects and with high selectivity. Additionally, the results are independent of the cell line. Likewise, EB1 increases the sensitivity of MDA-MB-231 tumour cells to chemotherapy when combined with doxorubicin (or abbreviated as DOXO in this description).

These results are especially surprising and relevant when other results from the derivatives studied with various substituents included in the phenyl rings at positions C4 and C6 of the 1*H*-pyrazolo[3,4-*b*]pyridin-3-amine system have presented activities lower than those of EB1.

The results of the radiometric enzyme assay performed by Proqinase^{®} (https://www.proqinase.com/) indicate that the residual activity of MNK1 and MNK2 kinases after treatment with 10 µM EB1 are 14 % and 52 %, respectively. These results indicate a certain selectivity for MNK1. In the same assay, the results obtained by the reference compounds cercosporamide and CGP57380 are, on one hand, -1 % and 0 % and, on the other, 21 % and 39 %.

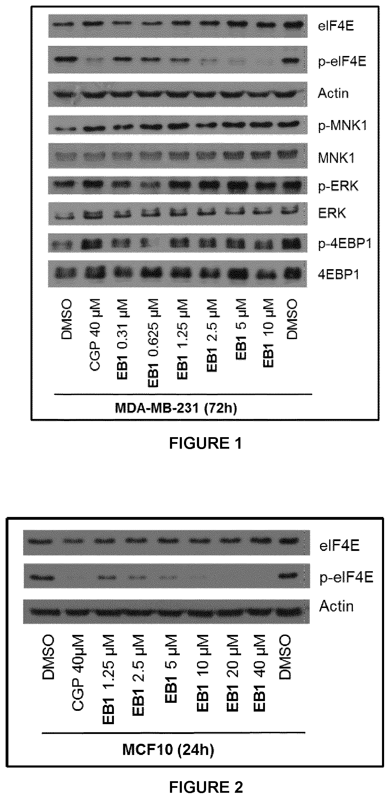

As shown in Figure 1, EB1 inhibits elF4E phosphorylation with *in vitro* ECso ~ 1.5 µM with almost complete inhibition as of 2.5 µM. The effect is fast and lasts for at least 72 h. Moreover, in the tested times it makes no difference whether the compound is renewed every 24 h or 48 h.

EB1 significantly reduces elF4E phosphorylation at concentrations greater than 2.5 µM. This reduction is not caused by a decrease in the total amount of elF4E according to Western Blot analysis. This effect seems to be due to the direct inhibition of MNKs, since the treatment does not affect any of the components of the signalling pathway (Figure 1).

EB1 does not alter the phosphorylation state of MNKs. Additionally, ERK, the kinase *downstream* of the MAP kinase signalling pathway, is not affected in control cells and cells treated with EB1.

Lastly, phosphorylation of the elF4E-binding protein (4E-BP1) is not altered by the compound either (Figure 1). In summary, the data indicates that decreased elF4E phosphorylation in cells treated with EB1 is due to the direct inhibition of MNK kinases. The effect of EB1 was also tested on immortalised non-tumour breast cells (MCF10) with similar results (EC₅₀ = 0.7 µM) (Figure 2).

Based on the fact that knockout mice of MNK1/2 are viable,and without being bound to any theory, it is proposed that both proteins are not essential for basic cellular functions. This is also supported by the fact that shRNA-mediated deletion of both proteins does not alter cell viability and growth. Based on this data, we predict that MNK inhibitors are not expected to reduce cell growth. Therefore, it is tested whether EB1 affects cell growth in concentrations, which are sufficient to inhibit elF4E phosphorylation.

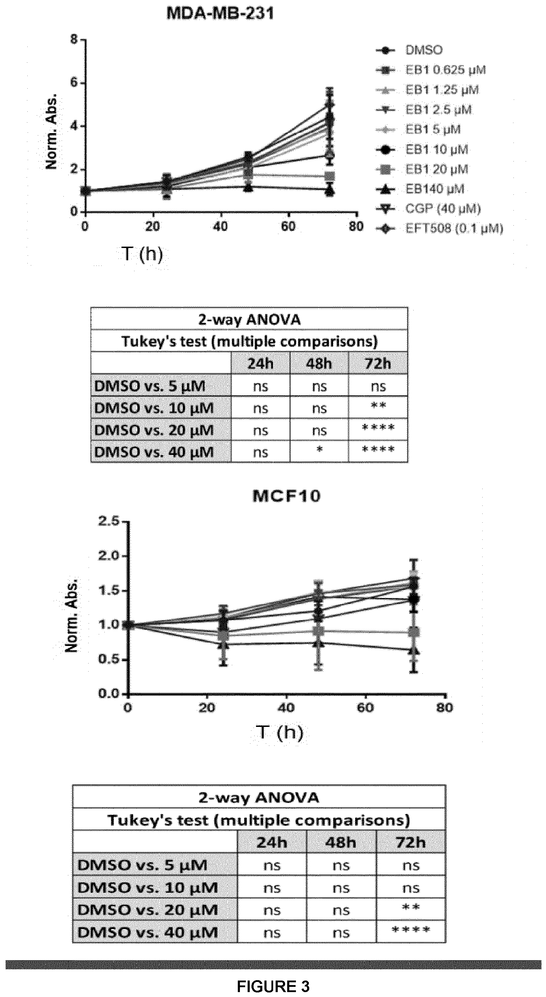

As shown in Figure 3, in TNBC cells (MDA-MB-231) and non-tumour breast cells (MCF10), it is possible to inhibit elF4E phosphorylation without altering cell growth and only observing a significant reduction in the number of cells at the highest concentrations.

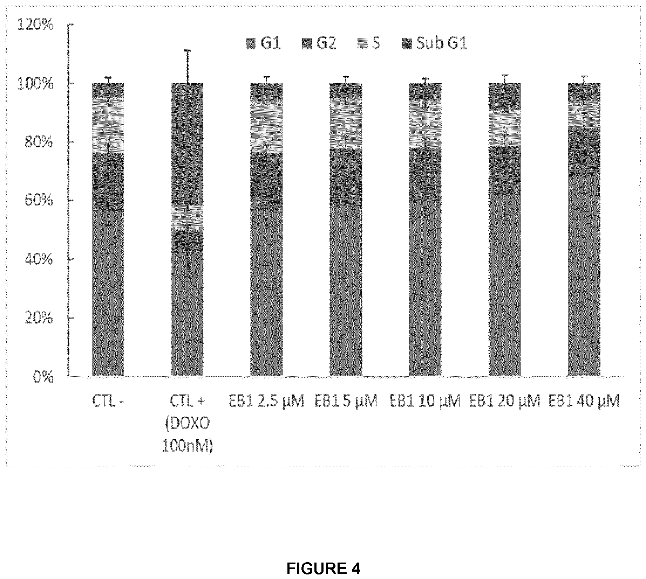

To determine how cell growth is affected at higher concentrations of EB1, a FACS analysis was performed to monitor the state of the cell cycle and the percentage of cells in subG1 as an indicator of cell death. As a positive control of cell death, cells were treated with 100 nM doxorubicin, which resulted in a sharp increase in cells in the subG1 phase. Unlike doxorubicin, concentrations up to 40 µM EB1 did not result in increased cell death. In contrast, an increase of cells could be observed in the G1 phase of the cell cycle, in a dose-dependent manner. These results, therefore, indicate that reduced cell growth at the highest concentrations of EB1 is caused by a delay in the cell cycle progression rather than by induction of cell death. Equivalent results have been described for the MNK1/2 inhibitor BAY 11433296 (Figure 4).

In summary, EB1 does not affect the cell growth of tumour and non-tumour cells at the concentrations necessary to inhibit MNK activity, which supports the selective mode of action towards MNK and provides a first indication of a safe mode of action in future clinical studies.

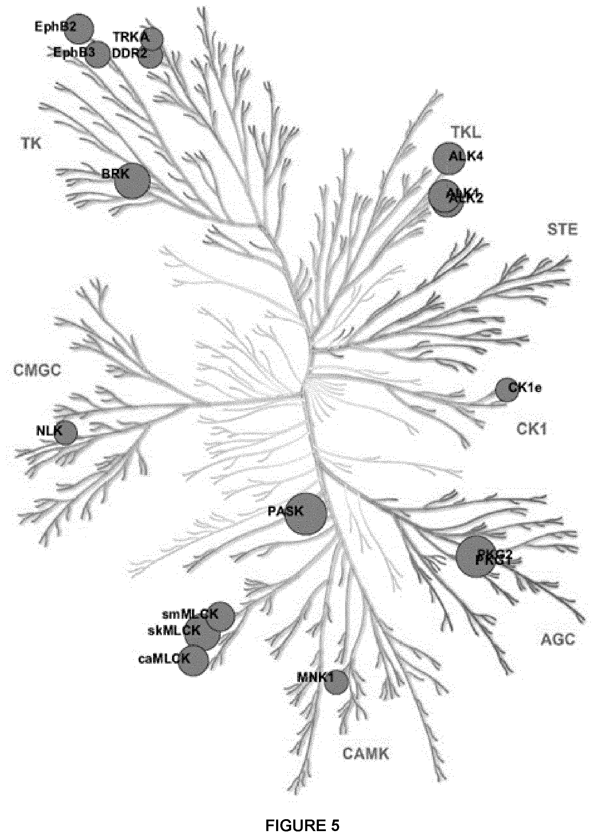

To check the selective mode of action, the activity of EB1 is assessed against a panel of different kinases (Proqinase^{®}) (Figure 5). EB1 shows excellent selectivity at 1 µM on a 320-kinase panel as only 16 other kinases are affected in an equivalent manner. A study of the literature reveals that none of the other affected kinases is involved in pathways that affect elF4E phosphorylation.

Reviews of previously described MNK inhibitors often describe specific effects for a given cell line. To test whether this is also the case for EB1, titration curves are performed on cell lines of different types of tumours, such as leukaemia cells (MV4-11), melanoma cells (A375M) and breast cancer cells (MDA-MB-468).

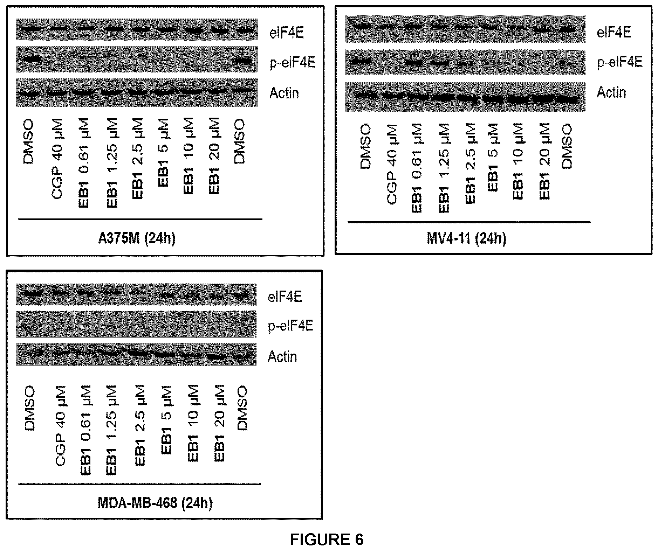

EB1 is active on all tested cell lines, which shows that the activity of said compound as an MNK inhibitor is not dependent on the cell line (Figure 6).

Inhibition of elF4E phosphorylation through MNK has emerged as a new strategy against cancer, especially in combination with other approved therapies. Treatment with MNK inhibitors makes it possible to sensitise tumour cells to common therapies. These new treatment schemes are also important for types of cancer in which targeted therapies are not available, such as triple-negative breast cancer. However, until now, no examples of the use of MNK inhibitors to sensitise triple-negative breast cancer to approved chemotherapies have been described.

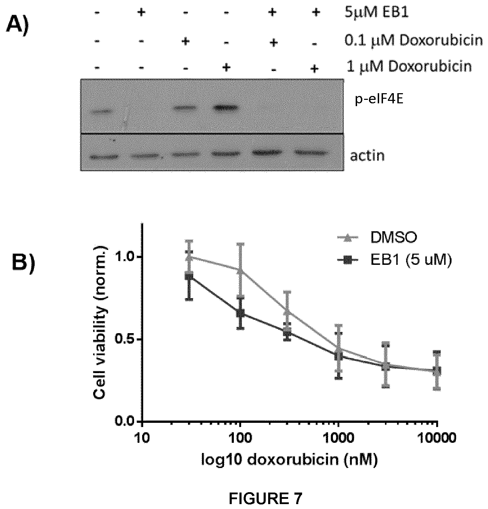

Combining EB1 with doxorubicin in MDA-MB-231 cells shows promising results. The increased elF4E phosphorylation caused by cellular stress due to treatment with doxorubicin has been confirmed and can be reversed with 5 µM EB1 (Figure 7A).

Additionally, the treatment together with EB1 sensitises MDA-MB-231 cells to doxorubicin, increasing the effect thereof on cell growth. The IC₅₀ of doxorubicin could be reduced in combination with EB1 from 350 nM to 225 nM (Figure 7B).

All these results confirm the use of 4,6-diphenyl-1*H*-pyrazolo[3,4-*b*]pyridin-3-amine (abbreviated as EB1), or of a pharmaceutically acceptable salt thereof, as a selective, non-cytotoxic inhibitor of MNK1 and MNK2.

### Example 3. EB1 is not causing adverse effects on cell growth and cell mortality in the IMR90 cell line as model for non-tumor cells.

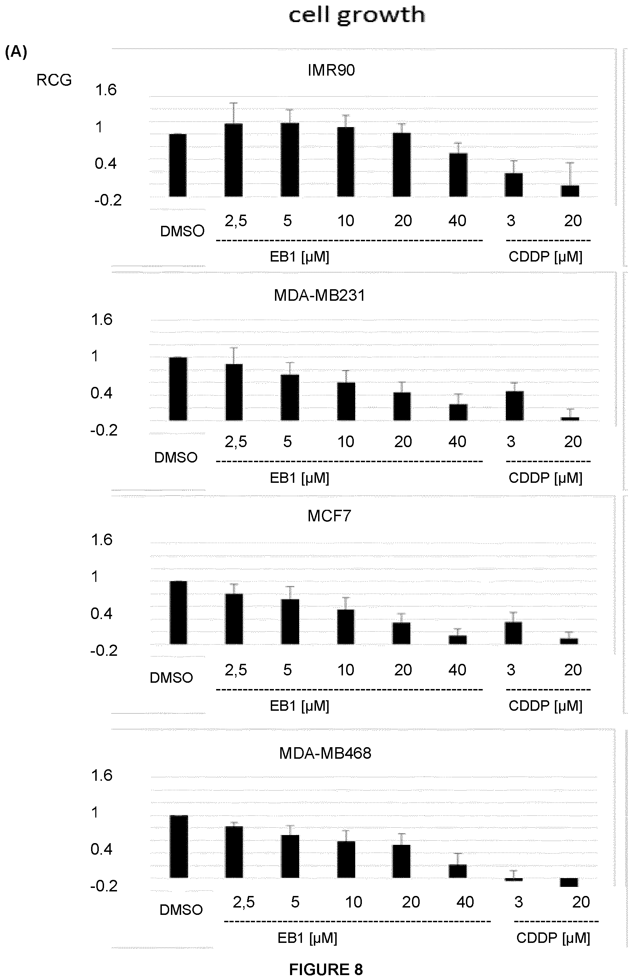

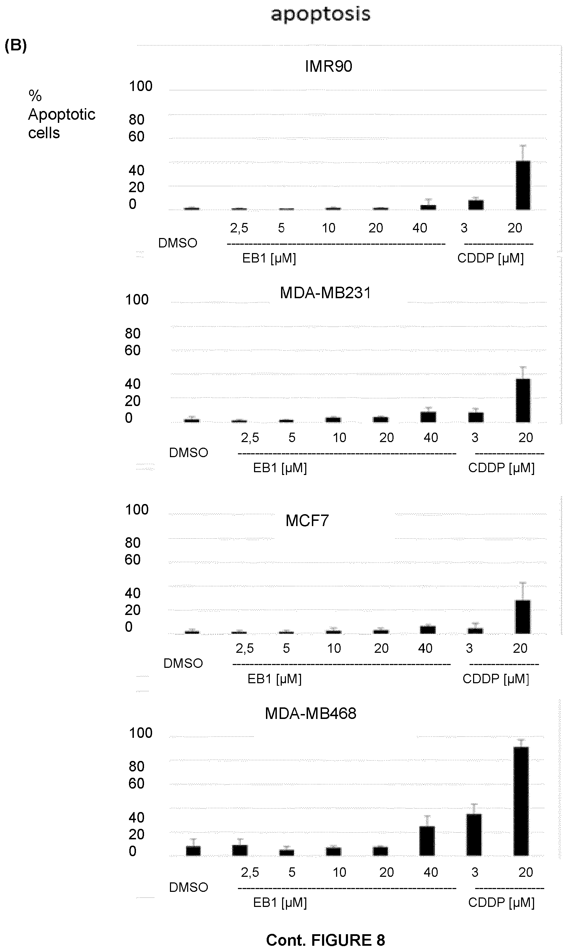

Knock-out mice of the EB1 target kinases MNK1 and MNK2 are viable (Ueda et al 2004; PMID: 15254222). Treatment of IMR90 cells, non-transformed fibroblasts, does not cause significant cell growth arrest (Figure 8, A) and does not induce cell death (Figure 8, B). Cell growth was measured by crystal violet assay. Cell death was determined by Propidium lodid (PI) / Hoechst staining and the ratio of dead cell (PI positive) among all analyzed cells (Hoechst) was determined. Cis-platin treatment was included as positive control. Results indicate that EB1 is not expected to cause adverse effects on healthy cells. Minimal side effects for patients are therefore predictable.

### Example 4. EB1 treatment results in increased sensitivity to tamoxifen

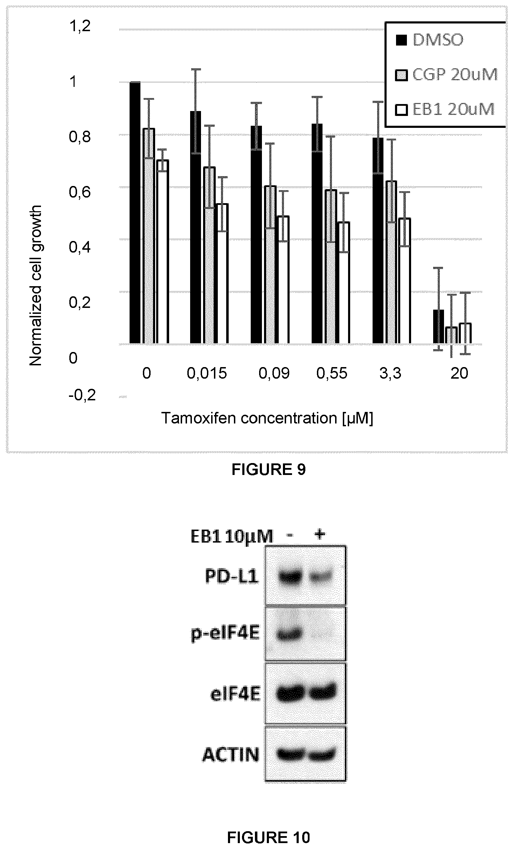

Previous studies suggest that the inhibition of MNK kinases by CGP57380 (CGP), and inhibitor that has a lot of targets, results in increased sensitivity to tamoxifen (Geter et al, 2017; PMID: 29269484) in MCF7 cells. EB1 treatment of MCF7 cells also results in increased sensitivity to tamoxifen (Figure 9). Cell growth in the presence of EB1, tamoxifen or the combination of both inhibitors was measured by crystal violet. Data were normalized to the vehicle control.

### Example 5. EB1 treatment results in reduced levels of PD-L1 in MDA-MB231 cells

Previous studies with eFT508 in syngenic mice models suggest that inhibition of MNKs results in reduced levels of PD-L1 expression. EB1 treatment reduces levels of PD-L1 in the human breast cancer cell line MDA-MB-231. At comparable inhibition rates of elF4E phosphorylation, EB1 is equally potent or superior to the MNK inhibitor eFT508 and CGP57380. MDA-MB-231 cells were treated for 48h with the indicated concentrations of the inhibitors. DMSO was used as vehicle control. Protein extracts were prepared and western blot analysis were carried out. Data are depicted in Figure 10.

### Example 6. EB1 treatment increases sensitivity to Enzalutamide in prostate cancer cells

Co-treatment of EB1 and Enzalutamide in the castration resistant prostate cancer cells line 22Rv1 (commercially at ATCC^{®} CRL-2505^{™}) resulted in a synergistic effect of the cell viability.

Cell viability under combined inhibition of AR and elF4E phosphorylation was assayed. Results are shown in Figure 11. In (A) Dose dependent inhibition of eIF4E phosphorylation was achieved after 24h EB1 treatment in 22Rv1, determined by western blot analysis. Synergistic effect of the EB1 and Enzalutamide was investigated with 22Rv1 (B-F) cell viability assay. (B) Number of viable cells after 72h treatment with pairwise combinations reported as percentage relative to control. (E) Cell viability data presented as a grid displaying the percentage of affected cells by each pairwise combination of drug doses. (C) Drug combinations with the strongest inhibition in cell viability (bars; mean ± SEM; n=3. *p<0.05, **p<0.01 two-tailed Student's test). (F) Combination index (CI) presented as a grid for each pairwise combination of drug doses calculated by the Chou-Talalay method at non-constant ratio. (D and I) Fraction affected vs. combination index (CI) plots.

All these data allow concluding that the combination comprising EB1 or a pharmaceutically or veterinary acceptable salt thereof and enzalutamide implies a synergistic effect in terms of reduction of cell viability of prostate cancer cells.

Dual inhibition of AR and elF4E phosphorylation induces cell death via apoptosis. These results are illustrated in Figure 12. With this aim, 22Rv1 (A) cells were treated either with vehicle (DMSO), enzalutamide, EB1 or a combination of both with the indicated concentrations for 72h. Whole cell lysates were then prepared and subjected to western blot analysis with the indicated antibodies. A reduction of AR, its splice variant AR-V7 and elF4E phosphorylation was observed upon treatment with EB1 alone and more pronounced with the combination. Increase in CI-PARP levels indicated apoptotic induction under the combinatory treatment. (B) Apoptosis assays of 22Rv1 cell line after treatment with the indicated concentrations of Enzalutamide, EB1 and the combination of both for 72h, quantified by analysis of fragmented/condensed chromatin after Propidium Iodide staining. Remarcable increase in apoptotic cell population was observed upon the dual treatment. Representative Propidium Iodide and Hoechst staining images of 22Rv1 cell line were obtained, although data are not shown. Data are mean of two technical replicates from one experiment ±SEM ***p<0.001 two-tailed Student's test

### Citation List

### Patent Literature

- US 2010/0113415
- WO 2011/019780
- WO 2015/004024
- DE 2160780

### Non Patent Literature

- Clin Cancer Res. 2007;13:4429-4434
- Nat Rev Clin Oncol. 2016;13:674-690
- Oncologist. 2011;16 Suppl 1:1-11
- Breast Cancer Res. 2010;12:212
- J. Am. Soc. Clin. Oncol. 2008;26:1275-1281
- Lancet Lond. Engl. 2014;384:164-172
- J. Am. Soc. Clin. Oncol. 2012;30:1796-1804
- Med. Oncol. Northwood Lond. Engl. 2017;35:13
- Clin Transl Oncol. 2014;16:937-1113.
- Oncotarget. 2015;6:24092-24104
- PLoS ONE. 2014;9:e89220
- J Cancer Res Clin Oncol. 2016;142:2309-2317
- J Neurooncol. 2016;131:485-493
- Virchows Arch. 2015;467:667-673
- Int J Clin Exp Pathol. 2015;8:3955-3962
- J Invest Dermatol. 2015;135:1358-1367
- Oncogene. 2015;34(16):2032-2042
- PLoS ONE. 2015;10(4):e0123352
- Mol Cell Biol. 2004;24(15):6539-6549
- Cancer Biol Ther. 2015;16(5):648-656
- Cancer Res. 2011;71:1849-1857
- Biochem. J. 2007;408(3):297-315
- Curr Med Chem. 2017;24(28):3025-3053.
- Oncotarget. 2012;3:118-131; Chem Biol. 2014;4:441-452
- Curr Med Chem. 2017;24(28):3025-3053
- J Med Chem. 2018;61(8):3516-3540
- Cancer Lett. 2017;390:21-29
- Geter et al, 2017; PMID: 29269484

## Claims

1. 4,6-diphenyl-1*H*-pyrazolo[3,4-*b*]pyridin-3-amine or a pharmaceutically or veterinary acceptable salt thereof, for use in the treatment of a cancer of an hormone-dependent organ selected from breast cancer, prostate cancer, ovarian cancer and endometrial cancer.

2. The 4,6-diphenyl-1H-pyrazolo[3,4-b]pyridin-3-amine, or a pharmaceutically or veterinary acceptable salt thereof, for use according to claim 1, wherein the cancer of an hormone-dependent organ is selected from breast cancer and prostate cancer.

3. The 4,6-diphenyl-1*H*-pyrazolo[3,4-*b*]pyridin-3-amine, or a pharmaceutically or veterinary acceptable salt thereof, for use according to claim 1, wherein the breast cancer is the triple negative breast cancer.

4. The 4,6-diphenyl-1*H*-pyrazolo[3,4-*b*]pyridin-3-amine, or a pharmaceutically or veterinary acceptable salt thereof, for use according to claim 2, wherein the cancer of an hormone-dependent organ is prostate cancer.

5. The 4,6-diphenyl-1*H*-pyrazolo[3,4-*b*]pyridin-3-amine, or a pharmaceutically or veterinary acceptable salt thereof, for use according to any one of claims 1-4, wherein the treatment comprises administering a therapeutically effective amount of 4,6-diphenyl-1*H-*pyrazolo[3,4-*b*]pyridin-3-amine, or a pharmaceutically or veterinary acceptable salt thereof, in combination with one or more compounds selected from compounds for chemotherapy and compounds for immunotherapy.

6. The 4,6-diphenyl-1*H*-pyrazolo[3,4-*b*]pyridin-3-amine, or a pharmaceutically or veterinary acceptable salt thereof, for use according to claim 5, wherein the chemotherapeutic compounds are selected from doxorubicin, tamoxifen, enzalutamide, docetaxel, cisplatin, lapatinib, and combinations thereof.

7. The 4,6-diphenyl-1*H*-pyrazolo[3,4-*b*]pyridin-3-amine, or a pharmaceutically or veterinary acceptable salt thereof, for use according to claim 5, wherein the compounds for immunotherapy comprise inhibitors of the programmed cell-death protein 1 and its ligand (PD-1/PD-L1).

8. A combination comprising:
a) a therapeutically effective amount of 4,6-diphenyl-1*H*-pyrazolo[3,4-*b*]pyridin-3-amine, or a pharmaceutically or veterinary acceptable salt thereof; and
b) a therapeutically effective amount of one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof;
wherein the combination is for use in the treatment of a cancer of an hormone-dependent organ selected from breast cancer, prostate cancer, ovarian cancer and endometrial cancer.

9. The combination for use according to claim 8, comprising 4,6-diphenyl-1*H-*pyrazolo[3,4-*b*]pyridin-3-amine, or a pharmaceutically or veterinary acceptable salt thereof, and one or more of enzalutamide and docetaxel.

10. The combination for use according to claim 8, comprising 4,6-diphenyl-1*H-*pyrazolo[3,4-*b*]pyridin-3-amine, or a pharmaceutically or veterinary acceptable salt thereof, and one or more of doxorubicin and tamoxifen.

11. The combination for use according to claim 8, comprising 4,6-diphenyl-1*H-*pyrazolo[3,4-*b*]pyridin-3-amine, or a pharmaceutically or veterinary acceptable salt thereof, and one or more of an inhibitor of the programmed cell-death protein 1 or its ligand (PD-1/PD-L1).

12. A single pharmaceutical or veterinary composition which comprises, together with one or more pharmaceutically or veterinary acceptable excipients or carriers, a therapeutically effective amount of:
a) 4,6-diphenyl-1*H*-pyrazolo[3,4-*b*]pyridin-3-amine, or a pharmaceutically or veterinary acceptable salt thereof; and
b) one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof;
wherein the single pharmaceutical or veterinary composition is for use in the treatment of a cancer of an hormone-dependent organ selected from breast cancer, prostate cancer, ovarian cancer and endometrial cancer.

13. A package or kit of parts comprising:
i) a first pharmaceutical or veterinary composition which comprises a therapeutically effective amount of 4,6-diphenyl-1*H*-pyrazolo[3,4-*b*]pyridin-3-amine, or a pharmaceutically or veterinary acceptable salt thereof, together with one or more pharmaceutically or veterinary acceptable excipients or carriers;
ii) a second pharmaceutical or veterinary composition which comprises a therapeutically effective amount of one or more chemotherapeutic or immunotherapeutic compounds selected from the group consisting of doxorubicin, tamoxifen, enzalutamide, docetaxel, cisplatin, lapatinib, inhibitors of the programmed cell-death protein 1 and its ligand (PD-1/PD-L1), and combinations thereof, together with one or more pharmaceutically or veterinary acceptable excipients or carriers;
iii) instructions for the use in combination of i) and ii);
wherein the first and second compositions are separate compositions;
wherein the package or kit of parts is for use in the treatment of a cancer of an hormone-dependent organ selected from breast cancer, prostate cancer, ovarian cancer and endometrial cancer.

## Patentansprüche

1. 4,6-Diphenyl-1*H*-pyrazolo[3,4-*b*]pyridin-3-amin oder ein pharmazeutisch oder tiermedizinisch akzeptables Salz davon zur Verwendung bei der Behandlung einer Krebserkrankung eines hormonabhängigen Organs, die ausgewählt aus Brustkrebs, Prostatakrebs, Eierstockkrebs und Endometriumkarzinom ist.

2. Das 4,6-Diphenyl-1H-pyrazolo[3,4-b]pyridin-3-amin, oder ein pharmazeutisch oder tiermedizinisch akzeptables Salz davon, zur Verwendung nach Anspruch 1, wobei die Krebserkrankung eines hormonabhängigen Organs aus Brustkrebs und Prostatakrebs ausgewählt ist.

3. Das 4,6-Diphenyl-1*H*-pyrazolo[3,4-*b*]pyridin-3-amin, oder ein pharmazeutisch oder tiermedizinisch akzeptables Salz davon, zur Verwendung nach Anspruch 1, wobei der Brustkrebs der triple-negative Brustkrebs ist.

4. Das 4,6-Diphenyl-1*H*-pyrazolo[3,4-*b*]pyridin-3-amin, oder ein pharmazeutisch oder tiermedizinisch akzeptables Salz davon, zur Verwendung nach Anspruch 2, wobei die Krebserkrankung eines hormonabhängigen Organs Prostatakrebs ist.

5. Das 4,6-Diphenyl-1*H*-pyrazolo[3,4-*b*]pyridin-3-amin, oder ein pharmazeutisch oder tiermedizinisch akzeptables Salz davon, zur Verwendung nach einem der Ansprüche 1 bis 4, wobei die Behandlung die Verabreichung einer therapeutisch wirksamen Menge von 4,6-Diphenyl-1*H*-pyrazolo[3,4-*b*]pyridin-3-amin, oder eines pharmazeutisch oder tiermedizinisch akzeptablen Salzes davon, in Kombination mit einer oder mehreren Verbindungen, ausgewählt aus Verbindungen für die Chemotherapie und Verbindungen für die Immuntherapie, umfasst.

6. Das 4,6-Diphenyl-1*H*-pyrazolo[3,4-*b*]pyridin-3-amin, oder ein pharmazeutisch oder tiermedizinisch akzeptables Salz davon, zur Verwendung nach Anspruch 5, wobei die chemotherapeutischen Verbindungen ausgewählt sind aus Doxorubicin, Tamoxifen, Enzalutamid, Docetaxel, Cisplatin, Lapatinib und Kombinationen davon.

7. Das 4,6-Diphenyl-1*H*-pyrazolo[3,4-*b*]pyridin-3-amin, oder ein pharmazeutisch oder tiermedizinisch akzeptables Salz davon, zur Verwendung nach Anspruch 5, wobei die Verbindungen für die Immuntherapie Inhibitoren des programmierten Zelltodproteins 1 und seines Liganden (PD-1/PD-L1) umfassen.

8. Eine Kombination, umfassend:
a) eine therapeutisch wirksame Menge von 4,6-Diphenyl-1*H*-pyrazolo[3,4-*b*]pyridin-3-amin, oder eines pharmazeutisch oder tiermedizinisch akzeptablen Salzes davon; und
b) eine therapeutisch wirksame Menge einer oder mehrerer chemotherapeutischer oder immuntherapeutischer Verbindungen, die ausgewählt sind aus der Gruppe bestehend aus Doxorubicin, Tamoxifen, Enzalutamid, Docetaxel, Cisplatin, Lapatinib, Inhibitoren des programmierten Zelltodproteins 1 und seines Liganden (PD-1/PD-L1) und Kombinationen davon;
wobei die Kombination zur Verwendung bei der Behandlung einer Krebserkrankung eines hormonabhängigen Organs bestimmt ist, die aus Brustkrebs, Prostatakrebs, Eierstockkrebs und Endometriumkarzinom ausgewählt ist.

9. Die Kombination zur Verwendung nach Anspruch 8, umfassend 4,6-Diphenyl-1*H-*pyrazolo[3,4-*b*]pyridin-3-amin, oder ein pharmazeutisch oder tiermedizinisch akzeptables Salz davon, und eines oder mehrere von Enzalutamid und Docetaxel.

10. Die Kombination zur Verwendung nach Anspruch 8, umfassend 4,6-Diphenyl-1*H-*pyrazolo[3,4-*b*]pyridin-3-amin, oder ein pharmazeutisch oder tiermedizinisch akzeptables Salz davon, und eines oder mehrere von Doxorubicin und Tamoxifen.

11. Die Kombination zur Verwendung nach Anspruch 8, umfassend 4,6-Diphenyl-1*H-*pyrazolo[3,4-*b*]pyridin-3-amin, oder ein pharmazeutisch oder tiermedizinisch akzeptables Salz davon, und einen oder mehrere Inhibitoren des programmierten Zelltodproteins 1 oder seines Liganden (PD-1/PD-L1).

12. Eine einzelne pharmazeutische oder tiermedizinische Zusammensetzung, welche zusammen mit einem oder mehreren pharmazeutisch oder tiermedizinisch akzeptablen Hilfsstoffen oder Trägersubstanzen eine therapeutisch wirksame Menge enthält von:
a) 4,6-Diphenyl-1*H*-pyrazolo[3,4-*b*]pyridin-3-amin, oder ein pharmazeutisch oder tiermedizinisch akzeptables Salz davon; und
b) eine oder mehrere chemotherapeutische oder immuntherapeutische Verbindungen, die aus der Gruppe ausgewählt sind, die aus Doxorubicin, Tamoxifen, Enzalutamid, Docetaxel, Cisplatin, Lapatinib, Inhibitoren des programmierten Zelltodproteins 1 und seines Liganden (PD-1/PD-L1) und Kombinationen davon besteht;
wobei die einzelne pharmazeutische oder tiermedizinische Zusammensetzung zur Verwendung bei der Behandlung einer Krebserkrankung eines hormonabhängigen Organs, die ausgewählt aus Brustkrebs, Prostatakrebs, Eierstockkrebs und Endometriumkarzinom ist, bestimmt ist.

13. Ein Paket oder ein Teilesatz, umfassend:
i) eine erste pharmazeutische oder tiermedizinische Zusammensetzung, welche eine therapeutisch wirksame Menge von 4,6-Diphenyl-1*H*-pyrazolo[3,4-*b*]pyridin-3-amin, oder eines pharmazeutisch oder tiermedizinisch akzeptablen Salzes davon, zusammen mit einem oder mehreren pharmazeutisch oder tiermedizinisch akzeptablen Hilfsstoffen oder Trägersubstanzen, umfasst;
ii) eine zweite pharmazeutische oder tiermedizinische Zusammensetzung, welche eine therapeutisch wirksame Menge einer oder mehrerer chemotherapeutischer oder immuntherapeutischer Verbindungen umfasst, die aus der Gruppe ausgewählt sind, die aus Doxorubicin, Tamoxifen, Enzalutamid, Docetaxel, Cisplatin, Lapatinib, Inhibitoren des programmierten Zelltodproteins 1 und seines Liganden (PD-1/PD-L1) und Kombinationen davon besteht, zusammen mit einem oder mehreren pharmazeutisch oder tiermedizinisch akzeptablen Hilfsstoffen oder Trägersubstanzen;
iii) Anweisungen für die Verwendung in Kombination von i) und ii);
wobei die erste und zweite Zusammensetzung getrennte Zusammensetzungen sind;
wobei die Verpackung oder der Teilesatz zur Verwendung bei der Behandlung einer Krebserkrankung eines hormonabhängigen Organs bestimmt ist, die aus Brustkrebs, Prostatakrebs, Eierstockkrebs und Endometriumkarzinom ausgewählt ist.

## Revendications

1. 4,6-diphényl-1*H*-pyrazolo[3,4-*b*]pyridin-3-amine ou un sel acceptable sur le plan pharmaceutique ou vétérinaire de celle-ci, destiné(e) à être utilisé(e) dans le traitement d'un cancer d'un organe hormono-dépendant choisi parmi le cancer du sein, le cancer de la prostate, le cancer de l'ovaire et le cancer de l'endomètre.

2. La 4,6-diphényl-1H-pyrazolo[3,4-b]pyridine-3-amine, ou un sel acceptable sur le plan pharmaceutique ou vétérinaire de celle-ci, destiné(e) à être utilisé(e) selon la revendication 1, dans laquelle le cancer d'un organe hormono-dépendant est choisi parmi le cancer du sein et le cancer de la prostate.

3. La 4,6-diphényl-1*H*-pyrazolo[3,4-*b*]pyridin-3-amine, ou un sel acceptable sur le plan pharmaceutique ou vétérinaire de celle-ci, destiné(e) à être utilisé(e) selon la revendication 1, dans laquelle le cancer du sein est le cancer du sein triple négatif.

4. La 4,6-diphényl-1*H*-pyrazolo[3,4-*b*]pyridin-3-amine, ou un sel acceptable sur le plan pharmaceutique ou vétérinaire de celle-ci, destiné(e) à être utilisé(e) selon la revendication 2, dans laquelle le cancer d'un organe hormono-dépendant est le cancer de la prostate.

5. La 4,6-diphényl-1*H*-pyrazolo[3,4-*b*]pyridine-3-amine, ou un sel acceptable sur le plan pharmaceutique ou vétérinaire de celle-ci, destiné(e) à être utilisé(e) selon l'une quelconque des revendications 1 à 4, dans laquelle le traitement comprend l'administration d'une quantité thérapeutiquement efficace de 4,6-diphényl-1*H-*pyrazolo[3,4-*b*]pyridine-3-amine, ou d'un sel acceptable sur le plan pharmaceutique ou vétérinaire de celle-ci, en combinaison avec un ou plusieurs composés choisis parmi les composés pour la chimiothérapie et les composés pour l'immunothérapie.

6. La 4,6-diphényl-1*H*-pyrazolo[3,4-*b*]pyridin-3-amine, ou un sel acceptable sur le plan pharmaceutique ou vétérinaire de celle-ci, destiné(e) à être utilisé(e) selon la revendication 5, dans laquelle les composés chimiothérapeutiques sont choisis parmi la doxorubicine, le tamoxifène, l'enzalutamide, le docétaxel, le cisplatine, le lapatinib et des combinaisons de ceux-ci.

7. La 4,6-diphényl-1*H*-pyrazolo[3,4-*b*]pyridin-3-amine, ou un sel acceptable sur le plan pharmaceutique ou vétérinaire de celle-ci, destiné(e) à être utilisé(e) selon la revendication 5, dans laquelle les composés pour l'immunothérapie comprennent des inhibiteurs de la protéine 1 de la mort cellulaire programmée et de son ligand (PD-1/PD-L1).

8. Une combinaison comprenant :
a) une quantité thérapeutiquement efficace de 4,6-diphényl-1*H*-pyrazolo[3,4-*b*]pyridine-3-amine, ou d'un sel acceptable sur le plan pharmaceutique ou vétérinaire de celle-ci ; et
b) une quantité thérapeutiquement efficace d'un ou plusieurs composés chimiothérapeutiques ou immunothérapeutiques choisis dans le groupe constitué de la doxorubicine, du tamoxifène, de l'enzalutamide, du docétaxel, du cisplatine, du lapatinib, des inhibiteurs de la protéine 1 de la mort cellulaire programmée et de son ligand (PD-1/PD-L1), et des combinaisons de ceux-ci ;
dans laquelle la combinaison est destinée à être utilisée dans le traitement d'un cancer d'un organe hormono-dépendant choisi parmi le cancer du sein, le cancer de la prostate, le cancer de l'ovaire et le cancer de l'endomètre.

9. La combinaison destinée à être utilisée selon la revendication 8, comprenant la 4,6-diphényl-1*H*-pyrazolo[3,4-*b*]pyridin-3-amine, ou un sel acceptable sur le plan pharmaceutique ou vétérinaire de celle-ci, et un ou plusieurs de l'enzalutamide et du docétaxel.

10. La combinaison destinée à être utilisée selon la revendication 8, comprenant la 4,6-diphényl-1*H*-pyrazolo[3,4-*b*]pyridin-3-amine, ou un sel acceptable sur le plan pharmaceutique ou vétérinaire de celle-ci, et un ou plusieurs de la doxorubicine et du tamoxifène.

11. La combinaison destinée à être utilisée selon la revendication 8, comprenant la 4,6-diphényl-1*H*-pyrazolo[3,4-*b*]pyridine-3-amine, ou un sel acceptable sur le plan pharmaceutique ou vétérinaire de celle-ci, et un ou plusieurs d'un inhibiteur de la protéine 1 de la mort cellulaire programmée ou de son ligand (PD-1/PD-L1).

12. Une composition pharmaceutique ou vétérinaire unique qui comprend, avec un ou plusieurs excipients ou véhicules acceptables sur le plan pharmaceutique ou vétérinaire, une quantité thérapeutiquement efficace de :
a) 4,6-diphényl-1*H*-pyrazolo[3,4-*b*]pyridin-3-amine, ou un sel acceptable sur le plan pharmaceutique ou vétérinaire de celle-ci ; et
b) un ou plusieurs composés chimiothérapeutiques ou immunothérapeutiques choisis dans le groupe constitué par la doxorubicine, le tamoxifène, l'enzalutamide, le docétaxel, le cisplatine, le lapatinib, les inhibiteurs de la protéine 1 de la mort cellulaire programmée et son ligand (PD-1/PD-L1), et des combinaisons de ceux-ci ;
dans laquelle la composition pharmaceutique ou vétérinaire unique est destinée à être utilisée dans le traitement d'un cancer d'un organe hormono-dépendant choisi parmi le cancer du sein, le cancer de la prostate, le cancer de l'ovaire et le cancer de l'endomètre.

13. Un emballage ou un kit de pièces comprenant :
i) une première composition pharmaceutique ou vétérinaire qui comprend une quantité thérapeutiquement efficace de 4,6-diphényl-1*H*-pyrazolo[3,4-*b*]pyridine-3-amine, ou d'un sel acceptable sur le plan pharmaceutique ou vétérinaire de celle-ci, avec un ou plusieurs excipients ou véhicules pharmaceutiquement ou vétérinaires acceptables ;
ii) une seconde composition pharmaceutique ou vétérinaire qui comprend une quantité thérapeutiquement efficace d'un ou plusieurs composés chimiothérapeutiques ou immunothérapeutiques choisis dans le groupe constitué de la doxorubicine, du tamoxifène, de l'enzalutamide, du docétaxel, du cisplatine, du lapatinib, des inhibiteurs de la protéine 1 de la mort cellulaire programmée et de son ligand (PD-1/PD-L1), et des combinaisons de ceux-ci, avec un ou plusieurs excipients ou véhicules acceptables sur le plan pharmaceutique ou vétérinaire ;
iii) des instructions destinées à être utilisées en combinaison de i) et ii) ;
dans lequel les première et seconde compositions sont des compositions séparées ;
dans lequel l'emballage ou le kit de pièces est destiné à être utilisé dans le traitement d'un cancer d'un organe hormono-dépendant choisi parmi le cancer du sein, le cancer de la prostate, le cancer de l'ovaire et le cancer de l'endomètre.
